# EUROPEAN PATENT SPECIFICATION

(11) **EP 4 151 278 B1**
(45) Date of publication and mention of the grant of the patent: **13.05.2026**
(21) Application number: 21788532.6
(22) Date of filing: 13.04.2021
(51) Int. Cl.: A61P 35/00, C07D 519/00, A61K 47/66, A61K 47/68, A61K 51/04, A61K 51/10, G01N 33/53, G01N 33/60

(54) **BIOTIN-MODIFIED DIMER AND USE THEREOF**
BIOTINMODIFIZIERTES DIMER UND SEINE VERWENDUNG
DIMÈRE MODIFIÉ PAR DE LA BIOTINE ET SON UTILISATION

(30) Priority: 14.04.2020 JP 2020072117
(43) Date of publication of application: 22.03.2023
(73) Proprietor: The University of Tokyo, Bunkyo-ku, Tokyo 113-8654 (JP); CompleCure, Inc., Tokyo 150-0002 (JP); Fukushima Medical University, Fukushima 960-1295 (JP)
(72) Inventor: KANAI Motomu, Tokyo 113-8654 (JP); YAMATSUGU Kenzo, Tokyo 113-8654 (JP); TATSUMI Toshifumi, Tokyo 113-8654 (JP); KODAMA Tatsuhiko, Tokyo 113-8654 (JP); SUGIYAMA Akira, Tokyo 113-8654 (JP); TSUKAGOSHI Masanobu, Tokyo 168-0064 (JP); TODA Yuzo, Tokyo 168-0064 (JP); WASHIYAMA Kohshin, Fukushima-shi, Fukushima 960-1295 (JP); TAKAHASHI Kazuhiro, Fukushima-shi, Fukushima 960-1295 (JP); ZHAO Songji, Fukushima-shi, Fukushima 960-1295 (JP); UKON Naoyuki, Fukushima-shi, Fukushima 960-1295 (JP); SUZUKI Miho, Fukushima-shi, Fukushima 960-1295 (JP)
(74) Representative: Godemeyer Blum Lenze Patentanwälte Partnerschaft mbB - werkpatent
(86) International application number: PCT/JP2021/015299
(87) International publication number: WO 2021/210573

(56) References cited:
- EP-A1- 3 109 252
- EP-A1- 3 584 576
- WO-A1-2015/125820
- WO-A1-2018/151301
- WO-A1-2019/189867
- WO-A1-2019/230905
- WO-A1-2020/032165
- JP-A- 2021 052 669
- US-A1- 2021 030 882
- US-A1- 2021 214 376
- US-A1- 2022 016 245
- TATSUMI TOSHIFUMI ET AL: "In vivo-stable bis-iminobiotin for targeted radionuclide delivery with the mutant streptavidin", BIOORGANIC & MEDICINAL CHEMISTRY LETTERS, vol. 108, 1 August 2024 (2024-08-01), Amsterdam NL, pages 129803, XP093321221, ISSN: 0960-894X, DOI: 10.1016/j.bmcl.2024.129803

## Description

### Technical Field

The present invention relates to a biotin-modified dimer and the use thereof.

### Background Art

Avidin and biotin, or streptavidin and biotin, have an extremely high affinity between them (Kd = 10⁻¹⁵ to 10⁻¹⁴ M). This is one of the extremely strong interactions between two biomolecules. Presently, the interaction between avidin/streptavidin and biotin has been widely applied in biochemistry, molecular biology, and medicine. A drug delivery method in which high binding ability between avidin/streptavidin and biotin is combined with an antibody molecule, namely, a pre-targeting method, has been devised.

Since chicken-derived avidin or microorganism-derived streptavidin exhibits high immunogenicity to a human body, such avidin or streptavidin is problematic in that an anti-avidin/streptavidin antibody is generated in an early stage after the administration thereof to a human body. This causes prevention of the practical use of the pre-targeting method. Low immunogenic streptavidin that solves the problem above has been reported (International Publication WO2010/095455).

Low immunogenic streptavidin is characterized by reduced immunogenicity to a human body. Since the low immunogenic streptavidin has an affinity for biotin existing in a human body, the low immunogenic streptavidin has been problematic in that it causes high background when used for diagnoses or in that it is not likely to exhibit medicinal effects specifically on a disease when used for treatments.

Under such circumstances, a mutant streptavidin with a reduced affinity for natural biotin and modified biotin having a high affinity for the mutant streptavidin with a reduced affinity for natural biotin have been reported (International Publication WO2014/129446). Moreover, a mutant streptavidin with a reduced affinity for natural biotin and a biotin-modified dimer having a high affinity for the mutant streptavidin with a low affinity for natural biotin have been reported (International Publication WO2015/125820). WO 2015/125820, WO 2020/032165, WO 2019/189867, WO 2018/151301 disclose biotin-modified dimers.

### Prior Art Documents

### Patent Documents

Patent Document 1: International Publication WO2010/095455
Patent Document 2: International Publication WO2014/129446
Patent Document 3: International Publication WO2015/125820

### Summary of Invention

### Object to be Solved by the Invention

It is an object of the present invention to provide a novel halogenated biotin-modified dimer capable of performing imaging diagnosis or treatment. Furthermore, it is another object of the present invention to provide a diagnostic kit and/or a therapeutic kit, in which a combination of the above-described biotin-modified dimer and a mutant streptavidin is used.

### Means for Solving the Object

Colorectal cancer has a high incidence rate and is on the increase. Even if colorectal cancer is stage 4, resection of distant metastasis is considered, and combination chemotherapy and molecular target drugs are used for this cancer. However, adjunctive therapy effective for vital prognosis has not yet been established. A majority of such colorectal cancers express carcinoembryonic antigen (CEA); thus, radio immunotherapy involving a specific anti-CEA antibody labeled with RI can be applied. In the present invention, it is an object of the invention to combine a drug delivery system (Cupid-Psyche system) with alpha-radionuclide astatine-211 (²¹¹At) so as to provide a novel radiopharmaceutical agent and a companion diagnostic agent for the treatment of refractory cancer.

Cupid-Psyche system is pre-targeting radio immunotherapy of utilizing a monoclonal antibody, to which a modified biotin (Psyche) and a tetrameric streptavidin (Cupid) bind, and this Cupid-Psyche system have been developed based on molecular dynamics simulation performed by a supercomputer.

In the Examples of the present invention, a treatment method of efficiently delivering ²¹¹At to colorectal cancer using the Cupid-Psyche system was developed. By using a Cupid-binding single chain variable fragment (scFv)-type antibody and ²¹¹At-labeled Psyche, the targeting of the Cupid-binding scFv-type antibody to the colorectal cancer cells was confirmed. At the same time, the stability of the ²¹¹At-labeled Psyche and the binding ability of the ²¹¹At-labeled Psyche to the Cupid-binding scFv-type antibody was confirmed. Moreover, the pharmacokinetics of Cupid-²¹¹At-labeled Psyche were analyzed, and the efficacy thereof in colorectal cancer animal models was evaluated. The present invention has been completed based on the above-described findings.

According to the present invention, the following inventions are provided.
<1> A compound represented by the following formula (1) or a salt thereof: 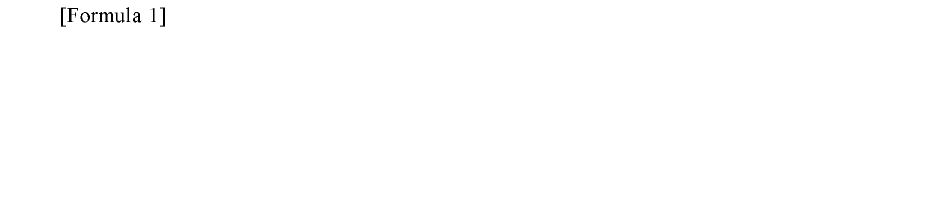 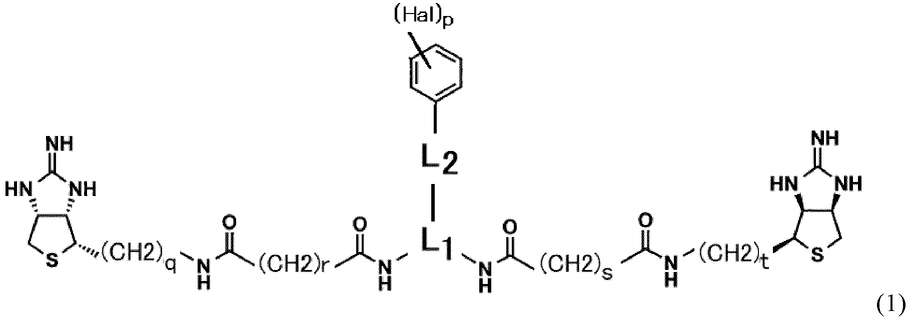 wherein
   L₁ represents a trivalent linking group,
   L₂ represents a divalent linking group,
   Hal represents a halogen,
   p represents an integer of 1 to 5, and
   q, r, s, and t each independently represent an integer of 1 to 8.
<2> The compound according to <1> or a salt thereof, wherein
   L₁ represents the following: 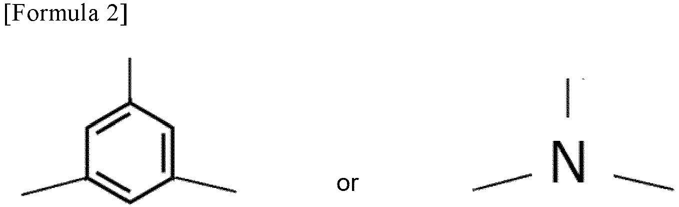
<3> The compound according to <1> or <2>, or a salt thereof, wherein
   L₂ represents a divalent linking group consisting of a combination of groups selected from -CONH-, -NHCO-, -COO-, -OCO-, -CO-, -O-, and an alkylene group containing 1 to 10 carbon atoms.
<4> The compound according to any one of <1> to <3>, or a salt thereof, which is a compound represented by the following formula (2) or a salt thereof: 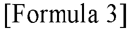 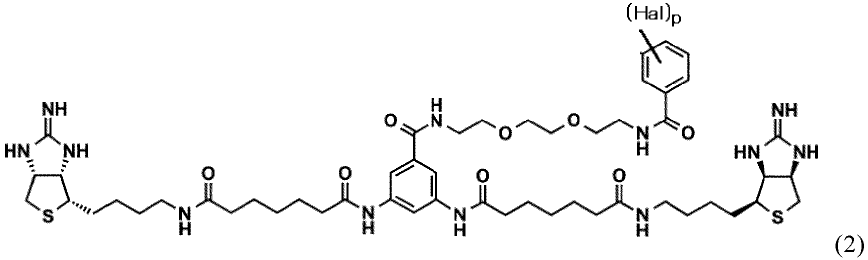 wherein individual symbols are as defined in claim 1.
<5> The compound according to any one of <1> to <4>, or a salt thereof, wherein Hal is I, ¹²⁵I, or ²¹¹At.
<6> The compound according to any one of <1> to <5>, or a salt thereof, wherein p is 1.
<7> Compounds represented by the following formulae: 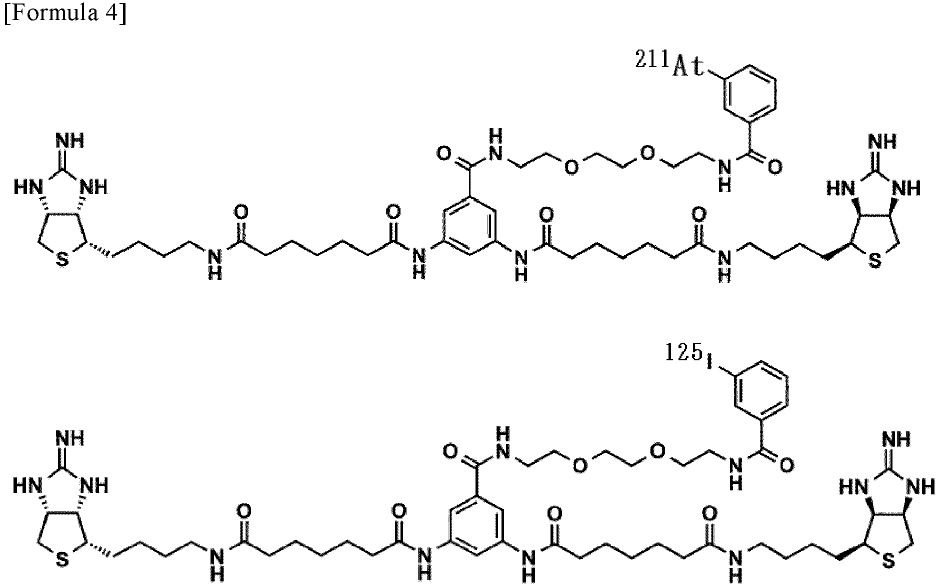 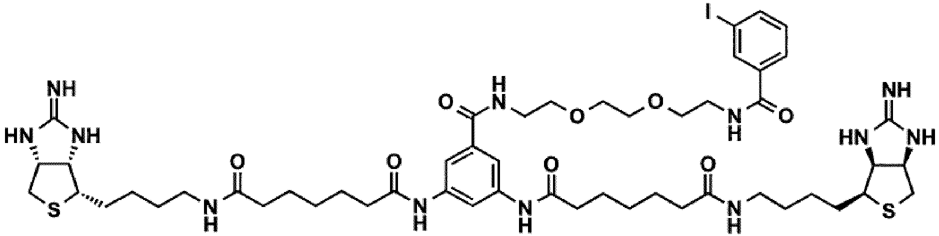
<8> A kit for treatment or diagnosis, comprising: (1) the compound according to any one of <1> to <7>; and (b) a mutant streptavidin-molecular probe conjugate obtained by binding a molecular probe to a mutant streptavidin comprising the amino acid sequence as set forth in SEQ ID NO: 1 (provided that the amino acid sequence consisting of six histidine residues at the C-terminus may be partially or entirely deleted).

### Advantageous Effects of Invention

The biotin-modified dimer, according to the present invention, and a kit comprising the same are functional in a diagnostic and/or therapeutic method based on the pre-targeting method.

### Brief Description of Drawings

[Fig. 1] Fig. 1 shows an outline of a domain structure.
[Fig. 2] Fig. 2 shows a CBB-stained SDS-PAGE electrophoretic pattern of CEA-V2122.
[Fig. 3] Fig. 3 shows the evaluation of the binding performance of CEA-Cupid with an antigen (CEACAM5).
[Fig. 4] Fig. 4 shows the evaluation of the binding performance of CEA-Cupid with modified biotin.
[Fig. 5] Fig. 5 shows the biodistribution (% ID/g) of a ¹²⁵I-Cupid-scFv antibody.
[Fig. 6] Fig. 6 shows the amount (% ID/g) of a ¹²⁵I-Cupid-scFv antibody accumulated in tumor tissues.
[Fig. 7] Fig. 7 shows the amount (% ID) of a ¹²⁵I-Cupid-scFv antibody accumulated in the thyroid gland, the tumor-to-blood ratio, and the tumor-to-muscle ratio.
[Fig. 8] Fig. 8 shows the biodistribution (% ID/g) of a Cupid-scFv antibody (24h before) + ²¹¹At-psyche-B.
[Fig. 9] Fig. 9 shows the accumulation (% ID) of a Cupid-scFv antibody (24h before) + ²¹¹At-psyche-B in the thyroid gland, the tumor-to-blood ratio, and the tumor-to-muscle ratio.
[Fig. 10] Fig. 10 shows the biodistribution (% ID/g) of a Cupid-scFv antibody (48h before) + ²¹¹At-psyche-B.
[Fig. 11] Fig. 11 shows the amount (% ID) of a Cupid-scFv (48h before) + ²¹¹At-psyche-B in the thyroid gland, the tumor-to-blood ratio, and the tumor-to-muscle ratio.
[Fig. 12] Fig. 12 shows the biodistribution (% ID/g) of ²¹¹At-psyche-B.
[Fig. 13] Fig. 13 shows the amount (% ID) of ²¹¹At-psyche-B in the thyroid gland.
[Fig. 14] Fig. 14 shows the biodistribution (% ID/g) of Cupid-scFv (24h before) + ²¹¹At-psyche-BR.
[Fig. 15] Fig. 15 shows the amount (% ID) of Cupid-scFv (24h before) + ²¹¹At-psyche-BR accumulated in the thyroid gland, the tumor-to-blood ratio, and the tumor-to-muscle ratio.
[Fig. 16] Fig. 16 shows the biodistribution (% ID/g) of Cupid-scFv (48h before) + ²¹¹At-psyche-BR.
[Fig. 17] Fig. 17 shows accumulation (% ID) of Cupid-scFv (48h before) + ²¹¹At-psyche-BR in the thyroid gland, the tumor-to-blood ratio, and the tumor-to-muscle ratio.
[Fig. 18] Fig. 18 shows the biodistribution (% ID/g) of ²¹¹At-psyche-BR.
[Fig. 19] Fig. 19 shows the accumulation (% ID) of ²¹¹At-psyche-BR in the thyroid gland.
[Fig. 20] Fig. 20 shows changes over time in mice's body weights (g) after the treatment with a Cupid-scFv-antibody + ²¹¹At-psyche-BR.
[Fig. 21] Fig. 21 shows changes over time in mice's tumor volumes (mm³) after the treatment with a Cupid-scFv-antibody + ²¹¹At-psyche-BR.
[Fig. 22] Fig. 22 shows the pharmacokinetics (% ID/g, 6h) of a Cupid-scFv antibody (24h before) + ²¹¹At-psyche-BR.
[Fig. 23] Fig. 23 shows changes over time in mice's body weights (g).
[Fig. 24] Fig. 24 shows changes over time in mice's tumor volumes (mm³).
[Fig. 25] Fig. 25 shows the pharmacokinetics (% ID/g, 6h) of a Cupid-scFv antibody (24h before) + ²¹¹At-psyche-B.

### Embodiments of Carrying out the Invention

Hereinafter, the present invention will be described in more detail.

### (1) Halogenated biotin-modified dimer

The present invention relates to a compound represented by the following formula (1) or a salt thereof, and preferably relates to a compound represented by the following formula (2) or a salt thereof. The compound of the present invention is also referred to as a "halogenated biotin-modified dimer."

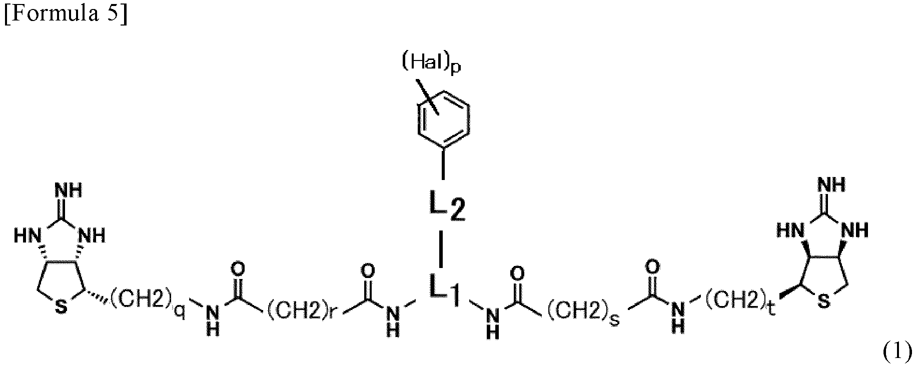

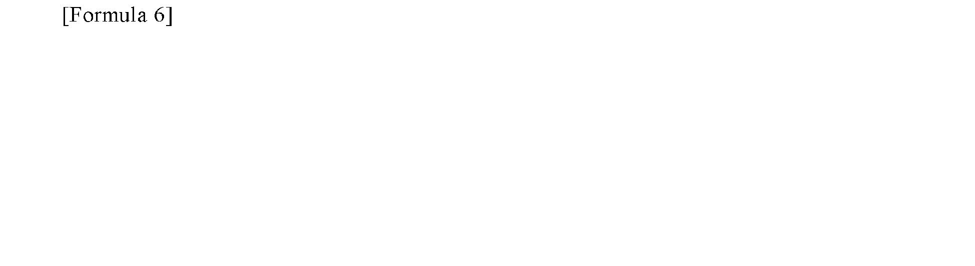

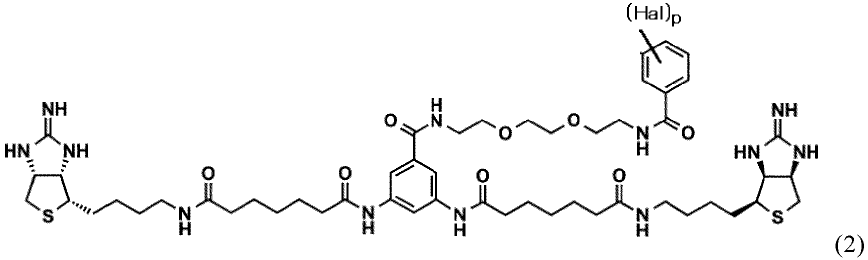

In the above formula, L₁ represents a trivalent linking group, and preferably represents the following: 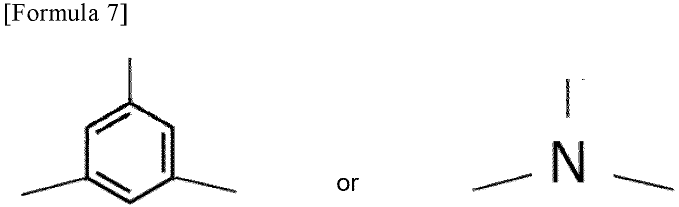 , and more preferably represents the following:

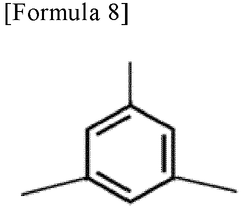

In the above formula, L₂ represents a divalent linking group.

L₂ is preferably a divalent linking group consisting of a combination of groups selected from -CONH-, -NHCO-, -COO-, -OCO-, -CO-, -O-, and an alkylene group containing 1 to 10 carbon atoms.

L₂ is preferably a divalent linking group consisting of a combination of groups selected from -CONH-, -NHCO-, -O-, and an alkylene group containing 1 to 10 carbon atoms.

Hal represents a halogen. Examples of the halogen represented by Hal may include fluorine (F), chlorine (Cl), bromine (Br), iodine (I), astatine (At), tennessine (Ts), and an isotope thereof. Preferred examples of the halogen may include iodine, astatine, and an isotope thereof. Preferred examples of the halogen may include ¹²³I, ¹²⁴I, ¹²⁵I, ¹³¹I, ²¹⁰At, and ²¹¹At. Among the above-described halogens, I, ¹²³I, ¹²⁴I, ¹²⁵I, ¹³¹I, and ²¹¹At are preferable. Hal is particularly preferably I, ¹²⁵I, or ²¹¹At.

p represents an integer of 1 to 5, preferably represents an integer of 1 to 3, and particularly preferably represents 1.

q, r, s, and t each independently represent an integer of 1 to 8, preferably independently represent an integer of 3 to 6, and particularly preferably represent 4 or 5.

Specific examples of the compound of the present invention may include the following compounds:

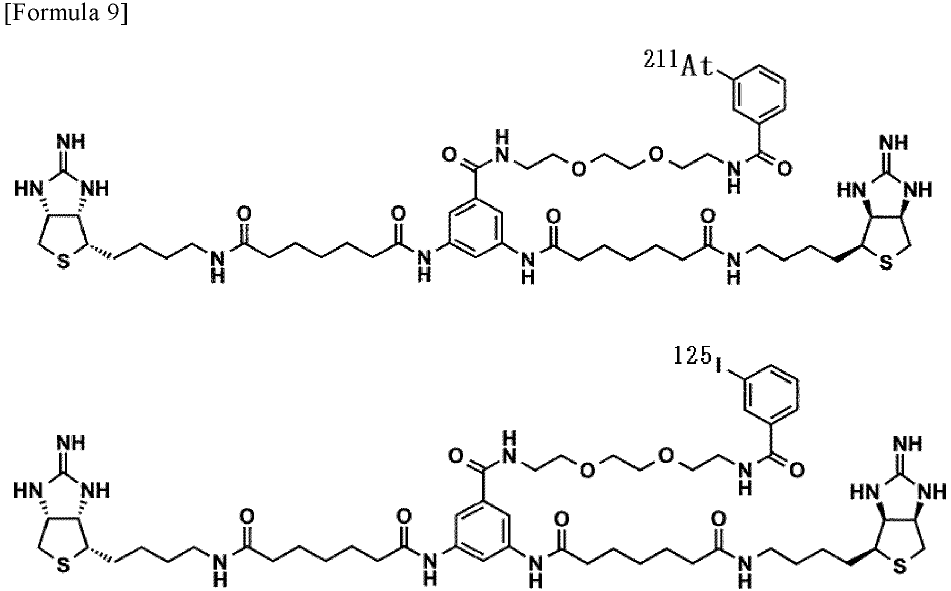

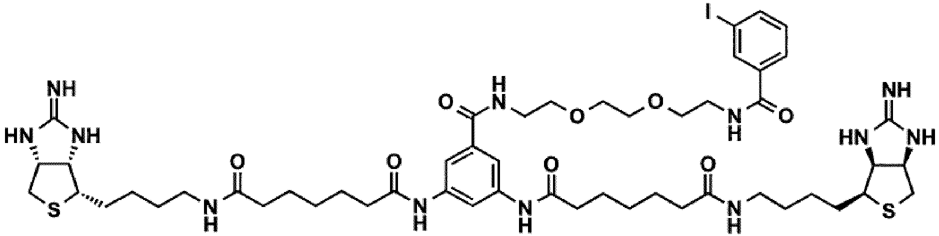

### (2) Method for producing halogenated biotin-modified dimer

As described in the Examples later, the halogenated biotin-modified dimer of the present invention can be produced by adding sodium halide (sodium iodide, Na¹²⁵I, etc.) or halogen (²¹¹At) dissolved in methanol to N-Bromo-succinimide in a mixed solution of methanol and acetic acid, then stirring the obtained mixture, then adding a compound represented by the following formula (A) dissolved in methanol to the reaction mixture, and then allowing the added compound to react with the reaction mixture.

The additive amount of *N*-Bromo-succinimide is preferably 0.1 to 1.0 equivalent, more preferably 0.1 to 0.5 equivalents, and further preferably 0.1 to 0.3 equivalents. The additive amount (equivalent) of *N*-Bromo-succinimide is preferably set to be larger than the additive amount of sodium halide or halogen. However, if the additive amount of *N-*Bromo-succinimide is excessively large, the compound represented by the following formula (A) is likely to be decomposed. Thus, the additive amount of *N*-Bromo-succinimide is preferably adjusted to be an appropriate amount. 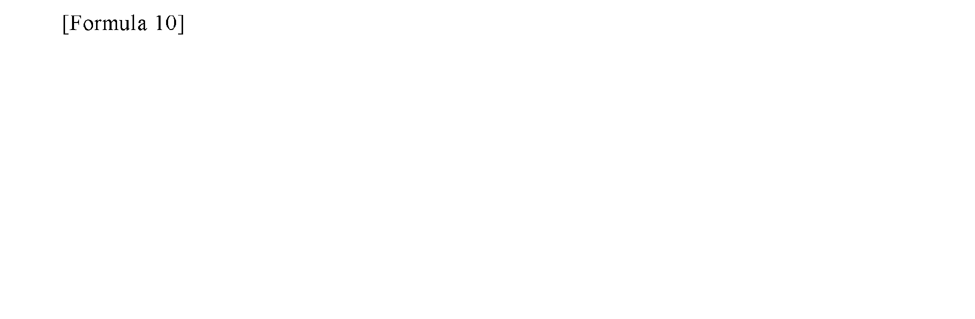 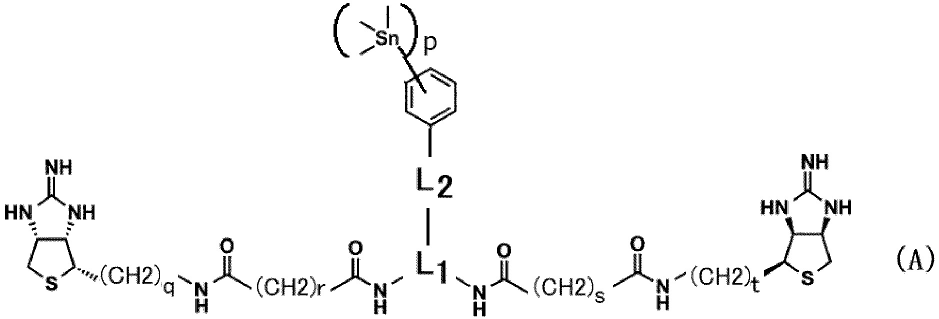 wherein each symbol is as defined in the formula (1).

### (3) Utilization of biotin-modified dimer

According to the present invention, provided is a therapeutic agent or a diagnostic agent, in which the biotin-modified dimer of the present invention is combined with a mutant streptavidin-molecular probe conjugate.

As mutant streptavidins used herein, the mutant streptavidins described in International Publication WO2014/129446 and International Publication WO2015/125820 can be used. Particularly preferably, the mutant streptavidin V2122 described in Example 3 of International Publication WO2015/125820 (SEQ ID NO: 4 of International Publication WO2015/125820) can be used.

Examples of the molecular probe used herein may include an antibody, a peptide, a nucleic acid, and an aptamer. Specifically, an antibody, a peptide, a nucleic acid, an aptamer, etc., which target the following antigens specifically expressed in cancer, can be used:

Epiregulin, ROBO 1, 2, 3, and 4, 1-40-β-amyloid, 4-1BB, 5AC, 5T4, ACVR2B, adenocarcinoma antigen, α-fetoprotein, angiopoetin 2, anthrax toxin, AOC3 (VAP-1), B-lymphoma cells, B7-H3, BAFF, β amyloid, C242 antigen, C5, CA-125, carbonic anhydrase 9 (CA-IX), cardiac myosin, CCL11 (eotaxin-1), CCR4, CCR5, CD11, CD18, CD125, CD140a, CD147 (basigin), CD147 (basigin), CD15, CD152, CD154 (CD40L), CD154, CD19, CD2, CD20, CD200, CD22, CD221, CD23 (IgE receptor), CD25 (IL-2 receptor α chain), CD28, CD3, CD30 (TNFRSF8), CD33, CD37, CD38 (cyclic ADP ribose hydrolase), CD4, CD40, CD41 (integrin α-IIb), CD44 v6, CD5, CD51, CD52, CD56, CD6, CD70, CD74, CD79B, CD80, CEA, CFD, ch4D5, CLDN18.2, *Clostridium difficile,* clumping factor A, CSF2, CTLA-4, cytomegalovirus, cytomegalovirus glycoprotein B, DLL4, DR5, *E. coli* Shiga toxin type 1, *E. coli* Shiga toxin type 2, EGFL7, EGFR, endotoxin, EpCAM, episialin, ERBB3, *Escherichia coli,* F protein of respiratory syncytial virus, FAP, fibrin II β chain, fibronectin extra domain-B, folate receptor 1, Frizzled receptor, GD2, GD3 ganglioside, GMCSF receptor α chain, GPNMB, hepatitis B surface antigen, hepatitis B virus, HER1, HER2/neu, HER3, HGF, HIV-1, HLA-DRβ, HNGF, Hsp90, human β amyloid, human scatter factor receptor kinase, human TNF, ICAM-1 (CD54), IFN-α, IFN-γ, IgE, IgE Fc region, IGF-1 receptor, IGF-I, IgG4, IGHE, IL-1β, IL-12, IL-13, IL-17, IL-17A, IL-22, IL-23, IL-4, IL-5, IL-6, IL-6 receptor, IL-9, ILGF2, influenza A hemagglutinin, insulin-like growth factor I receptor, integrin α4, integrin α4β7, integrin α5β1, integrin α7β7, integrin αIIbβ3, integrin αvβ3, integrin γ induced protein, interferon receptor, interferon α/β receptor, ITGA2, ITGB2 (CD18), KIR2D, L-selectin (CD62L), Lewis-Y antigen, LFA-1 (CD11a), lipoteichoic acid, LOXL2, LTA, MCP-1, mesothelin, MS4A1, MUC1, mucin CanAg, myostatin, N-glycolylneuraminic acid, NARP-1, NCA-90 (granulocyte antigen), NGF, NOGO-A, NRP1, Oryctolagus cuniculus, OX-40, oxLDL, PCSK9, PD-1, PDCD1, PDGF-R α, phosphatidylserine, prostate cancer cells, *Pseudomonas aeruginosa,* Rabies virus glycoprotein, RANKL, respiratory syncytial virus, RHD, Rh (Rhesus) factor, RON, RTN4, sclerostin, SDC1, selectin P, SLAMF7, SOST, sphingosine-1-phosphate, TAG-72, TEM1, tenascin C, TFPI, TGFβ1, TGFβ2, TGF-β, TNF-α, TRAIL-R1, TRAIL-R2, tumor antigen CTAA16.88, MUC1 tumor-specific glycosylation, TWEAK receptor, TYRP1 (glycoprotein 75), VEGF-A, VEGFR-1, VEGFR2, vimentin, and VWF.

A fusion body of a molecular probe such as a tumor antigen-specific antibody molecule and a mutant streptavidin is prepared, and the prepared fusion body is then administered to a patient, so that the mutant streptavidin can be accumulated specifically in cancer cells. Subsequently, the halogenated biotin-modified dimer of the present invention having an affinity for the above-described mutant streptavidin is administered to the patient, so that the halogen can be accumulated exactly in the cancer cells. In the present invention, the generation of an antibody is suppressed by a reduction in immunogenicity, and thereby, the clearance of the mutant streptavidin from the body in an early stage caused by the antibody, or shock such as anaphylaxis, can be prevented. Moreover, in the present invention, using the mutant streptavidin of the present invention as an in-vitro diagnostic agent or a clinical diagnostic agent, regarding which the tissue, serum and the like collected from patients are used, noise derived from biotin or a biotin-binding protein present in the tissue, serum and the like can be reduced, so that diagnosis and examination with a higher S/N ratio can be carried out.

Otherwise, in the present invention, a conjugate is prepared by binding a fusion body of a molecular probe such as a tumor antigen-specific antibody molecule and a mutant streptavidin with the halogenated biotin-modified dimer of the present invention, and the thus prepared conjugate can be administered to a patient.

Various types of molecules can be used as antibodies that are to be bound to the mutant streptavidin. Either a polyclonal antibody or a monoclonal antibody may be used. The subclass of the antibody is not particularly limited. Preferably, IgG, and particularly preferably, IgG₁ is used. Furthermore, the term "antibody" includes all of modified antibodies and antibody fragments. Examples of such an antibody include, but are not limited to: a humanized antibody; a human type antibody; a human antibody; antibodies from various types of animals such as a mouse, a rabbit, a rat, a guinea pig and a monkey; a chimeric antibody between a human antibody and an antibody from a different type of animal; diabody; scFv; Fd; Fab; Fab'; and F(ab)'₂.

The conjugate of the mutant streptavidin and the antibody can be obtained by applying a method known to persons skilled in the art. For example, such a conjugate can be obtained by a chemical bond method (US5,608,060). Alternatively, DNA encoding the mutant streptavidin is ligated to DNA encoding an antibody, and using an expression vector or the like, the ligated DNA is then expressed in a host cell, so that such a conjugate can be obtained in the form of a fusion protein. The DNA encoding the mutant streptavidin may be ligated to the DNA encoding an antibody via DNA encoding a suitable peptide, called a linker. The mutant streptavidin-molecular probe conjugate is desirably produced, while keeping the specific binding force between an antibody and a target molecule.

The present invention will be more specifically described in the following examples. However, these examples are not intended to limit the scope of the present invention.

### Examples

### < Synthesis of biotin-modified dimer compound >

### < General Methods >

Nuclear magnetic resonance (NMR) spectra were measured using a JEOL ECX500 (¹H NMR: 500MHz) or JEOL ECS400 (¹H NMR: 400MHz) spectrometer. Chemical shift was expressed in ppm as a value with respect to the residual solvent peak in a deuterated solvent as an internal reference (CDCl₃:δ = 7.26 ppm, CD₃OD:δ = 3.31 ppm). Low-resolution mass spectra (LHMS) were measured using ESI-MS according to Shimadzu LCMS-2020 System. The reaction was traced by thin-layer chromatography (TLC) or low-resolution mass spectrometry (LRMS).

Reverse phase high performance liquid chromatography (HPLC) was carried out using JASCO-HPLC System. Detection was conducted using ultraviolet ray at a wavelength of 254 nm, and a gradient solvent system (acetonitrile/0.1% trifluoroacetic acid MQ solution or 0.1% formic acid MQ solution) was used as a mobile phase. The analysis was carried out using a YMC-Triart-C18 (150 mm x 4.6 mm I.D.) column at a flow rate of 1 mL/min. Fractionation was carried out using a YMC-Triart-C18 (250 mm x 10 mm I.D. or 150 mm x 4.6 mm I.D.) column at a flow rate of 6.3 mL/min in the case of the former column or at a flow rate of 1 mL/min in the case of the latter column.

Methyl 5-((2*S*,3*S*,4*R*)-3,4-diaminotetrahydrothiophen-2-yl)pentanoate hydrobromide (2)

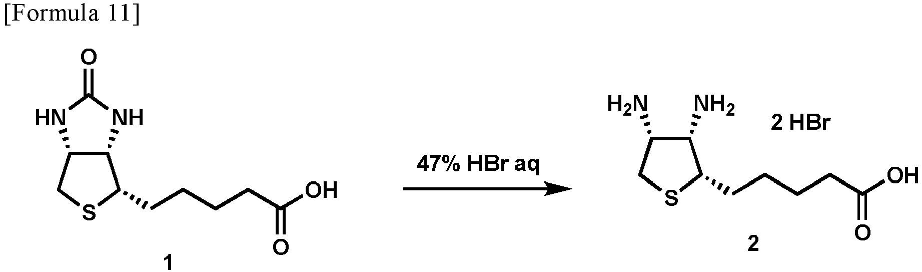

A 47% hydrogen bromide aqueous solution (24 ml) was added to biotin 1 (5 g, 20.4 mmol), and the obtained mixture was then stirred under reflux in an argon atmosphere at 150°C for 90 hours. Thereafter, the solvent was removed under reduced pressure. The obtained crude product 2 was used in the subsequent reaction, without being subjected to further purification operations.

5-((2*S*,3*S*,4*R*)-3,4-Bis((*tert*-butoxycarbonyl)amino)tetrahydrothiophen-2-yl)pentanoic acid (3)

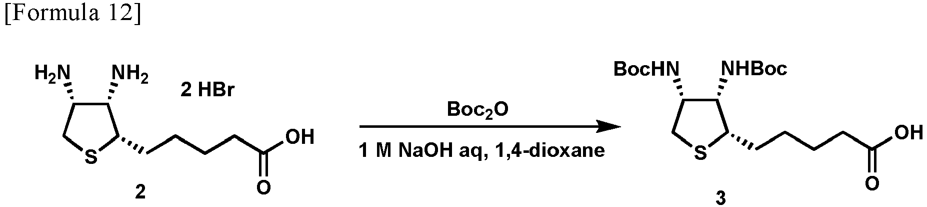

Di-tert-butyl dicarbonate (12.8 g, 61.3 mmol) was added to the crude product 2 in a mixed solution consisting of 1,4-dioxane (100 ml) and a 1 M NaOH aqueous solution (100 ml) at room temperature. The obtained mixture was stirred in an argon atmosphere at room temperature for 22 hours. Thereafter, the solvent was removed under reduced pressure, and a 1 M HCl aqueous solution was then added to the residue, followed by extraction with chloroform. The organic layer was washed with brine. The resultant was dried over sodium sulfate, and the solvent was then removed under reduced pressure. The obtained crude product 3 was used in the subsequent reaction, without being subjected to further purification operations.

Di-*tert-*butyl ((2*S,*3*S,*4*R*)-2-(4-aminobutyl)tetrahydrothiophene-3,4-diyl)dicarbamate (4)

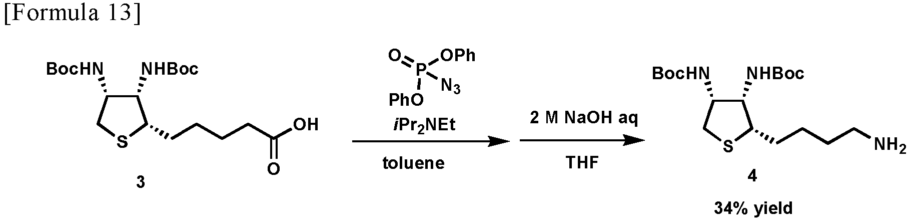

*N,N*-Diisopropylethylamine (293 µl, 1.70 mmol) and diphenylphosphoryl azide (367 µl, 1.70 mmol) were added to the crude product 3 (471 mg, 1.13 mmol) in a toluene (6 ml) solution at room temperature, and the obtained mixture was then stirred in an argon atmosphere at 85°C for 3 hours 40 minutes. Thereafter, *N,N-*diisopropylethylamine (98 µl, 0.73 mmol) and diphenylphosphoryl azide (122 µl, 0.73 mmol) were further added to the reaction mixture, and the thus obtained mixture was then stirred at 85°C for 1 hour. The reaction mixture was cooled to room temperature, and THF (7 ml) and a 2 M NaOH aqueous solution (4 ml) were then added thereto. The obtained mixture was stirred at room temperature for 13 hours, and the solvent was then removed under reduced pressure. The obtained crude product was purified by silica gel chromatography (chloroform/methanol = 95 : 5 → 75 : 25 → 65 : 35) to obtain the target compound 4 (151 mg, yield: 34%, 3 steps).
¹H NMR (500 MHz, CDCl₃) δ: 1.28-1.72 (m, 24H), 2.47 (t, *J =* 10.3 Hz, 1H), 2.66 (t, *J* = 7.4 Hz, 2H), 3.16-3.28 (m, 1H), 3.44-3.60 (m, 1H), 4.16-4.40 (m, 2H), 4.72-4.88 (m, 1H), 4.90-5.08 (m, 1H). LRMS (ESI): *m*/*z* 390.95 [M+H]⁺.

Benzyl 7-((4-((2*S*,3*S*,4*R*)-3,4-bis((*tert*-butoxycarbonyl)amino)tetrahydrothiophen-2-yl)butyl)amino)-7-oxoheptanoate (5)

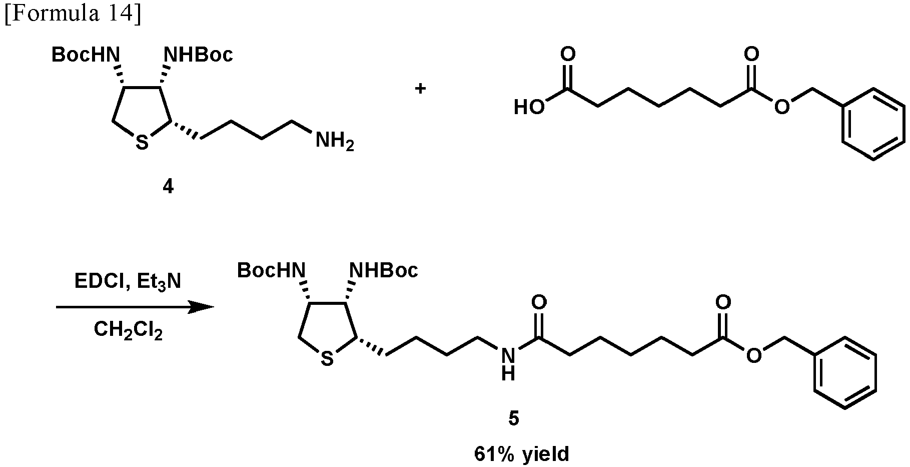

Triethylamine (115 µl, 0.825 mmol) and EDCI (157 mg, 0.825 mmol) were added to the compound 4 (129.4 mg, 0.33 mmol) and 7-(benzyloxy)-7-oxoheptanoic acid (Reference 1) (150 mg, 0.599 mmol) in a dichloromethane (5ml) solution at room temperature. The obtained mixture was stirred in an argon atmosphere at room temperature for 18 hours. Subsequently, the solvent was removed under reduced pressure, and water was then added to the residue, followed by extraction with ethyl acetate. After that, the organic layer was successively washed with a saturated sodium bicarbonate solution, a saturated ammonium chloride aqueous solution, and brine. The resultant was dried over sodium sulfate, and the solvent was then removed under reduced pressure. The obtained crude product was purified by silica gel chromatography (chloroform/methanol = 100 : 0 → 95 : 5) to obtain the target compound 5 (126 mg, yield: 61%).
¹H NMR (500 MHz, CDCl₃) δ: 1.26-1.70 (m, 30H), 2.15 (t, *J =* 6.9 Hz, 2H), 2.36 (t, *J* = 7.4 Hz, 2H),2.46 (t, *J =* 9.7 Hz, 1H), 3.11-3.29 (m, 3H), 3.41-3.53 (m, 1H), 4.16-4.27 (m, 1H), 4.28-4.36 (m, 1H), 4.72-4.81 (m, 1H),4.89-4.98 (m, 1H), 5.11 (s, 2H), 5.55-5.65 (m, 1H), 7.28-7.40 (m, 5H). LRMS (ESI): *m*/*z* 644.10 [M+Na]⁺.

Benzyl 7-((4-((2*S*,3*S*,4*R*)-3,4-diaminotetrahydrothiophen-2-yl)butyl)amino)-7-oxoheptanoate trifluoroacetate (6)

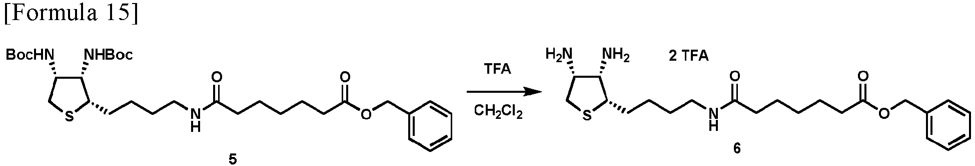

Trifluoroacetic acid (1 ml) was added to the compound 5 (126 mg, 0.203 mmol) in a dichloromethane (4 ml) solution. The obtained mixture was stirred in an argon atmosphere at room temperature for 2 hours. Thereafter, the solvent was removed under reduced pressure, and toluene azeotrope was then performed three times. After that, trifluoroacetic acid was removed under reduced pressure. The crude product 6 was used in the subsequent reaction, without being subjected to further purification operations.

Benzyl 7-((4-((3a*S*,4*S*,6a*R*,*E*)-2-((*tert*-butoxycarbonyl)imino)hexahydro-1*H*-thieno[3,4-d]imidazol-4-yl)butyl)amino)-7-oxoheptanoate (7)

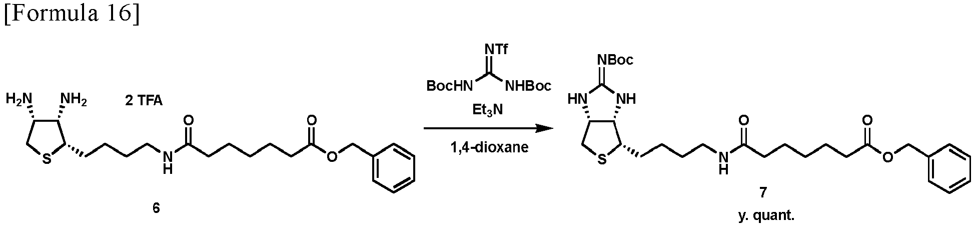

Goodman reagent (83.4 mg, 0.213 mmol) dissolved in 1,4-dioxane (1.5 ml) was added to the compound 6 (0.203 mmol) in a mixed solution consisting of a 1,4-dioxane solution (2 ml) and triethylamine (141 µl, 1.02 mmol) at room temperature. The obtained mixture was stirred in an argon atmosphere at 50°C for 2 hours, and the solvent was then removed under reduced pressure. The obtained crude product was purified by silica gel chromatography (chloroform/methanol = 100 : 0 → 93 : 7) to obtain the target compound 7 (yield: quant., 2 steps). ¹H NMR (500 MHz, CDCl₃) δ: 1.22-1.78 (m, 21H), 2.15 (t, *J =* 7.4 Hz, 2H), 2.35 (t, *J =* 7.4 Hz, 2H), 2.88 (d, *J* = 13.2 Hz, 1H), 3.01 (dd, *J = 4.6* Hz, 13,2 Hz, 1H), 3.10-3.20 (m, 1H), 3.21-3.28 (m, 1H), 3.29-3.38 (m, 1H), 4.60 (dd, *J = 4.6* Hz, 8.6 Hz, 1H), 4.79 (dd, *J* = 4.6 Hz, 8.0 Hz, 1H), 5.10 (s, 2H), 7.30-7.40 (m, 5H). LRMS (ESI): *m*/*z* 547.25 [M+H]⁺.

7-((4-((3a*S*,4*S*,6a*R*,*E*)-2-((*tert*-Butoxycarbonyl)imino)hexahydro-*1*H-thieno[3,4-d]imidazol-4-yl)butyl)amino)-7-oxoheptanoic acid (8)

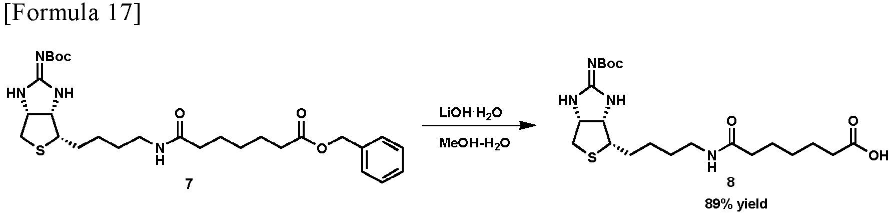

Lithium hydroxide hydrate (21.3 mg, 0.51 mmol) was added to the compound 7 (0.203 mmol) in a mixed solution consisting of methanol (3 ml) and water (1.5 ml) at room temperature. The obtained mixture was stirred in an argon atmosphere at 50°C for 1 hour 20 minutes, and the solvent was then removed under reduced pressure. After that, a saturated ammonium chloride aqueous solution was added to the residue, followed by extraction with chloroform. The extract was dried over sodium sulfate, and the solvent was then removed under reduced pressure. The obtained crude product was purified by silica gel chromatography (chloroform/methanol = 99 : 1 → 85 : 15 → 80 : 20 → 75 : 25) to obtain the target compound 8 (83.2 mg, yield: 89%, white solid).
¹H NMR (500 MHz,CD₃OD) δ: 1.26-1.87 (m, 21H), 2.19 (t, *J =* 6.8 Hz, 2H), 2.22 (t, *J =* 7.4 Hz, 2H), 2.89 (d, *J* = 13.2 Hz, 1H),3.02 (dd, *J* = 4.6 Hz, 13.2 Hz, 1H), 3.10-3.29 (m, 3H), 4.53 (dd, *J = 4.6* Hz, 8.0 Hz, 1H), 4.76 (dd, *J* = 4.6 Hz, 8.6 Hz, 1H). LRMS (ESI): *m*/*z* 457.85 [M+H]⁺.

Di-*tert*-butyl (5-((3-oxo-1-phenyl-2,7,10-trioxa-4-azadodecan-12-yl)carbamoyl)-1,3-phenylene)dicarbamate (10)

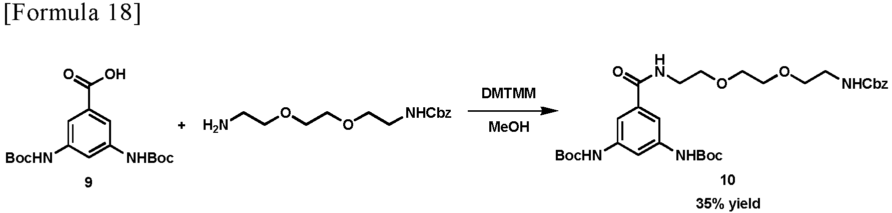

Benzyl (2-(2-(2-aminoethoxy)ethoxy)ethyl)carbamate (Reference 3) (646 mg, 2.29 mmol) and 4-(4,6-dimethoxy-1,3,5-triazin-2-yl)-4-methylmorpholinium Chloride (DMTMM, 530 mg, 1.92 mmol) were added to the compound 9 (Reference 2) (450 mg, 1.28 mmol) in a methanol (5 ml) solution at room temperature. The obtained mixture was stirred in an argon atmosphere at room temperature for 115 hours, and the solvent was then removed under reduced pressure. After that, a 1 M HCl aqueous solution was added to the residue, followed by extraction with ethyl acetate, and the organic layer was successively washed with a saturated sodium bicarbonate solution and brine. The resultant was dried over sodium sulfate, and the solvent was then removed under reduced pressure. The obtained crude product was purified by silica gel chromatography (chloroform/methanol = 96:4) to obtain the target compound 10 (273 mg, yield: 35%, yellow solid).
¹H NMR (400 MHz, CDCl₃) δ: 1.49 (s, 18H), 3.32-3.47 (m, 2H), 3.54-3.70 (m, 10H), 5.07 (s, 2H), 5.36-5.47 (m, 1H), 6.68-6.86 (m, 2H), 6.88-6.98 (m, 1H), 7.28-7.40 (m, 5H), 7.42-7.51(m, 2H), 7.67-7.77 (m, 1H).
LRMS (ESI): *m*/*z* 617.05 [M+H]⁺.

Benzyl (2-(2-(2-(3,5-diaminobenzamido)ethoxy)ethoxy)ethyl)carbamate trifluoroacetate (11)

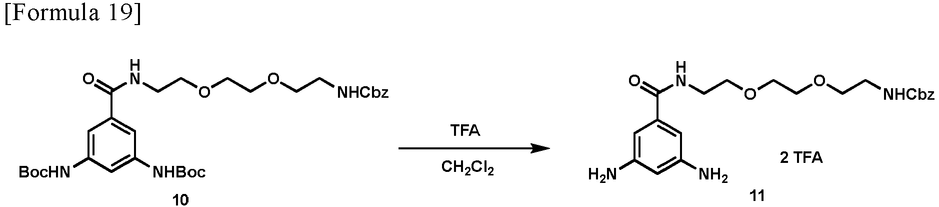

Trifluoroacetic acid (0.5 ml) was added to the compound 10 (86.8 mg, 0.14 mmol) in a dichloromethane (4.5 ml) solution at room temperature. The obtained mixture was stirred in an argon atmosphere at room temperature for 4 hours, and the solvent was then removed under reduced pressure. After that, toluene azeotrope was performed twice, and trifluoroacetic acid was then removed under reduced pressure. The crude product 11 was used in the subsequent reaction, without being subjected to further purification operations.

*tert*-Butyl ((3a*S*,4*S*,6a*R,E*)-4-(4-(7-((3-(7-((4-((3a*S*,4*S*,6a*R,Z*)-2-((*tert-*butoxycarbonyl)imino)hexahydro-1*H*-thieno[3,4-d]imidazol-4-yl)butyl)amino)-7-oxoheptanamido)-5-((3-oxo-1-phenyl-2,7,10-trioxa-4-azadodecan-12-yl)carbamoyl)phenyl)amino)-7-oxoheptanamido)butyl)tetrahydro-1*H*-thieno[3,4-d]imidazol-2(3H)-ylidene)carbamate (12)

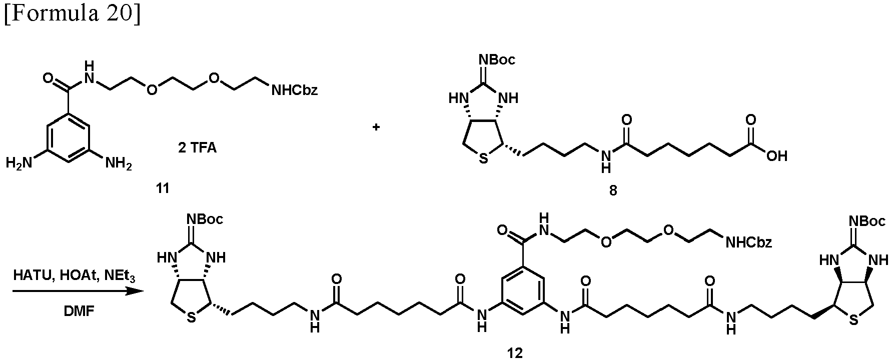

1-Hydroxy-7-azabenzotriazole (HOAt, 32 mg, 235 µmol) and 1-[Bis(dimethylamino)methyliumyl]-1*H*-1,2,3-triazolo[4,5-b]pyridine-3-oxide hexafluorophosphate (HATU, 89.4 mg, 235 µmol) were added to the compound 11 (37.9 mg, 58.8 µmol) and the compound 8 (67.3 mg, 147 µmol) in a mixed solution consisting of DMF (1 ml) and triethylamine (65.6 µl, 470 µmol) at room temperature. The obtained mixture was stirred in an argon atmosphere at room temperature for 16 hours. Thereafter, the solvent was removed under reduced pressure, and water was then added to the residue, followed by extraction with chloroform. The extract was dried over sodium sulfate, and the solvent was then removed under reduced pressure. The obtained residue was purified by silica gel chromatography (chloroform/methanol = 97 : 3 → 75 : 25 → 40 : 60) to obtain a roughly purified product comprising the target compound 12 (20.5 mg).
¹H NMR (400 MHz, CD₃OD) δ1.21-1.90 (m, 42H), 2.19 (t, *J =* 7.2 Hz, 4H), 2.37 (t, *J* = 7.2 Hz, 4H), 2.92 (d, *J =* 13.0 Hz, 2H), 3.01 (dd, *J =* 4.5 Hz, 13.1 Hz, 2H), 3.05-3.67 (m, 18H), 4.51-4.60 (m, 2H), 4.75-85 (m, 2H), 5.03 (s, 2H), 7.23-7.36 (m, 5H), 7.67-7.73 (m, 2H), 7.98-8.05(m, 1H). LRMS (ESI): *m*/*z* 647.90 [M+2H]²⁺.

*N1,N1'*-(5-((2-(2-(2-Aminoethoxy)ethoxy)ethyl)carbamoyl)-1,3-phenylene)bis(*N7*-(4-((3a*S*,4*S*,6a*R*)-2-iminohexahydro-1*H*-thieno[3,4-d]imidazol-4-yl)butyl)heptanediamide) 2,2,2-trifluoroacetate (13)

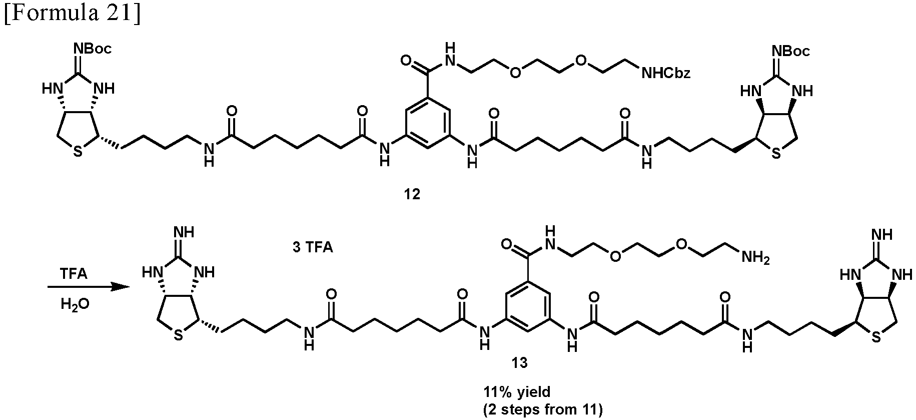

Trifluoroacetic acid (3 ml) was added to an aqueous solution (150 µl) of the compound 12 (20.5mg, 15.8 µmol) at room temperature. The obtained mixture was stirred in an argon atmosphere at room temperature for 14 hours 30 minutes, and the solvent was then removed under reduced pressure. The obtained crude product was purified by reverse phase HPLC (gradient: 0% for 5 min; 2%-100% for 90 min CH₃CN in 0.1% CF₃COOH aqueous solution, retention time: 35.2 min, YMC-Triart C18, flow rate = 3.5 ml/min) to obtain the target compound 13 (8.3 mg, yield: 11%, 2 steps).
¹H NMR (400 MHz, CD₃OD) δ: 1.35-1.81 (m, 24H), 2.20 (t, *J =* 7.6 Hz, 4H), 2.39 (t, *J =* 7.2 Hz, 4H), 2.81 (d, *J* = 13.5 Hz, 2H), 2.98 (dd, *J =* 4.9 Hz, 13.0 Hz, 2H), 3.07-3.17 (m, 4H), 3.18-3.28 (m, 4H), 3.57 (t, *J* = 6.3 Hz, 2H), 3.64-3.72 (m, 8H), 4.50 (dd, *J* = 4.5 Hz, 8.1 Hz, 2H), 4.71 (dd, *J = 4.5* Hz, 8.1 Hz, 2H), 7.76 (d, *J = 2.2* Hz, 2H), 7.90 (t, *J =* 1.8 Hz, 1H). LRMS (ESI): *m*/*z* 320.65 [M+3H]³⁺.

*N1,N1'*-(5-((2-(2-(2-(3-(Trimethylstannyl)benzamido)ethoxy)ethoxy)ethyl)carbamoyl)-1,3-phenylene)bis(N7-(4-((3a*S*,4*S*,6a*R*)-2-iminohexahydro-1*H*-thieno[3,4-d]imidazol-4-yl)butyl)heptanediamide) formate (15)

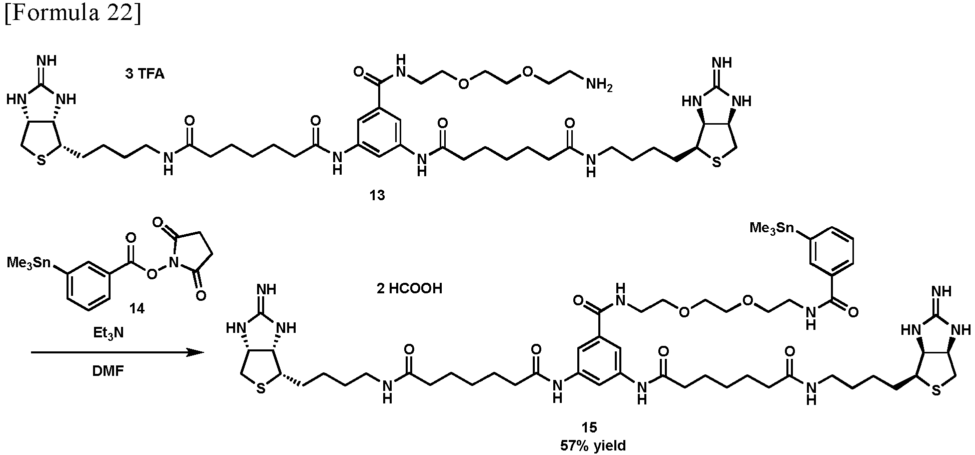

The compound 14 (Reference 4) (1.9 mg, 5.0 µmol) and triethylamine (9.5 µl, 68 µmol) were added to the compound 13 (5.9 mg, 4.5 µmol) in a DMF (400 µl) solution at room temperature. The obtained mixture was stirred in an argon atmosphere at room temperature for 3 hours 30 minutes, and the solvent was then removed under reduced pressure. The obtained crude product was purified by reverse phase HPLC (gradient: 0% for 5 min; 2%-100% for 90 min CH₃CN in 0.1% HCOOH aqueous solution, retention time: 40.5 min, YMC-Triart C18, flow rate = 3.5 ml/min) to obtain the target compound 15 (3.4 mg, yield: 57%).
LRMS (ESI): *m*/*z* 614.10 [M+2H]²⁺.

### References

1. Su, Z.; Yeagley, A. A.; Su, R.; Peng, L.; Melander, C. ChemMedCmem 2012, 7, 2030-2039
2. Roy, B. C.; Malik, S. J. Org. Chem. 199, 64, 2969-2974.
3. Thoma, G.; Streiff, M. B.; Katapodis, A. G.; Duthaler, R. O.; Voelcker, N. H.; Ehrhardt, C.; Masson, C. Chem.-Eur. J. 2006, 12, 99-117.
4. GE HEALTHCARE LIMITED, Michelle, A.; Jane, B.; Peter, B. I. WO2010/86398, 2010, A1.

### < Labeling with ²¹¹At >

²¹¹At (10-150 MBq) dissolved in methanol(100 µl) was added to *N-*Bromosuccinimide (0.00271 mg, 0.152 µmol) in a mixed solution consisting of methanol (100 µl) and acetic acid (1 µl), and the obtained mixture was then stirred at room temperature for 1 minute. Thereafter, bisiminobiotin (compound 15) (0.1 mg, 0.076 µmol) dissolved in methanol (100 µl) was added to the reaction mixture, and the thus obtained mixture was then stirred at room temperature for 10 minutes. Thereafter, 800 µl of water was added to the reaction solution to dilute it, and the thus diluted solution was then purified by reverse phase HPLC (20% for 2 min; 20%-70% for 20 min CH₃CN in 0.1% CF₃COOH aqueous solution, retention time = 9.7 min, YMC-Triart C18, flow rate = 4.7 ml/min ) to obtain a ²¹¹At-labeled compound 23 (also referred to as "²¹¹At-psyche-BR").

Structure of ²¹¹At-labeled compound 23 (²¹¹At-psyche-BR):

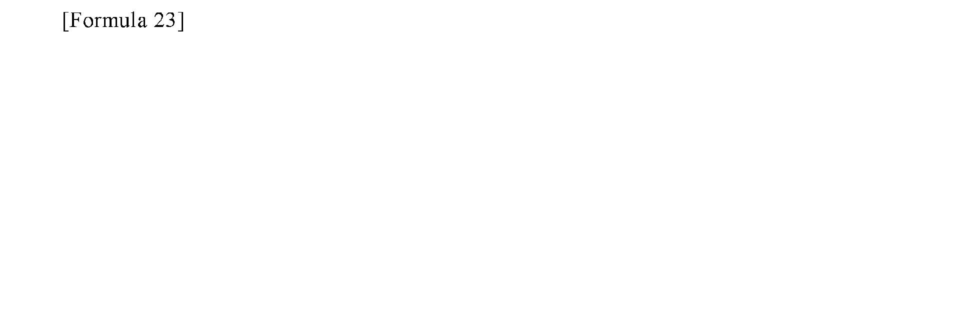

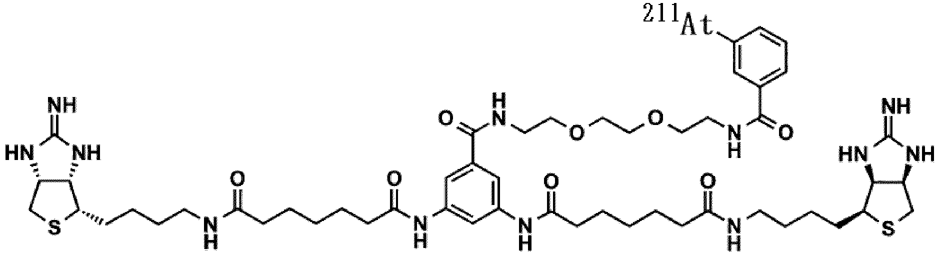

### Regarding the production of ²¹¹At-psyche-B

A compound 24 having the structure shown below was synthesized as described in Example 1 of International Publication WO2019/230905.
Structure of compound 24 (bisiminobiotin 24 (Psyche-B-ATE)):

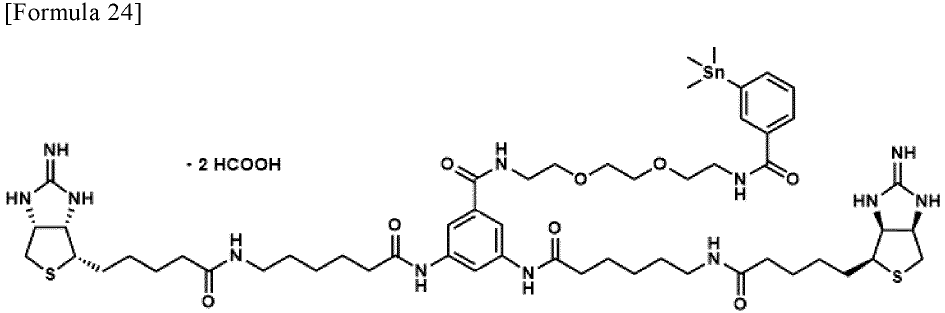

### < Labeling with ²¹¹At >

²¹¹At (10-150 MBq) dissolved in methanol (100 µl) was added to *N-*Bromosuccinimide (0.00271 mg, 0.152 µmol) in a mixed solution consisting of methanol (100 µl) and acetic acid (1 µl), and the obtained mixture was then stirred at room temperature for 1 minute. Thereafter, bisiminobiotin (compound 24) (0.1 mg, 0.076 µmol) dissolved in methanol (100 µl) was added to the reaction mixture, and the thus obtained mixture was then stirred at room temperature for 10 minutes. Thereafter, 800 µl of water was added to the reaction solution to dilute it, and the thus diluted solution was then purified by reverse phase HPLC (20% for 2 min; 20%-70% for 20 min CH₃CN in 0.1% CF₃COOH aqueous solution, retention time = 9.7 min, YMC-Triart C18, flow rate = 4.7 ml/min ) to obtain a ²¹¹At-labeled compound 25 (also referred to as "²¹¹At-psyche-B").

Structure of ²¹¹At-labeled compound 25 (²¹¹At-Psyche-B):

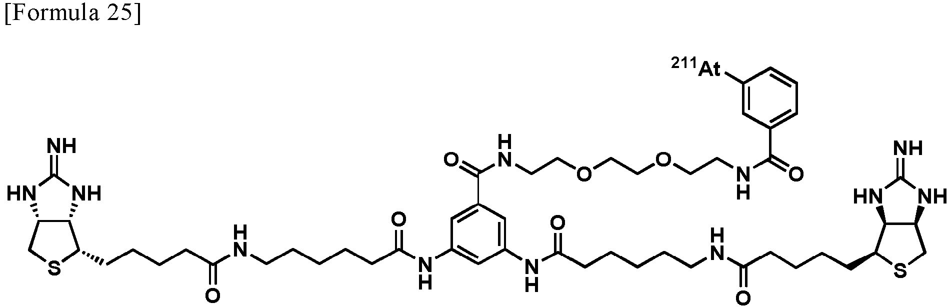

The recovered solution of the compounds 23 and 25 recovered by reverse phase HPLC was 2-fold diluted by addition of a 0.1% TFA solution, and was then passed through a Sep-Pak solid phase extraction column (Sep-pak C18 light; manufactured by Waters). A 0.5% Na ascorbate solution (6 mL) was supplied to the Sep-Pak column, so that unnecessary solvents and contaminants were washed away from the column, and a product of interest was recovered with 1 mL of PBS solution containing 50% ethanol, 0.5% acetic acid, and 0.5% Na ascorbate.

### < Preparation of Cupid-scFv antibody >

### (1) Expression and purification of CEA-V2122 protein (also referred to as a "Cupid-scFv antibody")

V2122 is the mutant streptavidin described in Example 3 of International Publication WO2015/125820 (SEQ ID NO: 4 shown in International Publication WO2015/125820). The amino acid sequence of V2122 (a sequence having a 6xHis tag at the C-terminus) is as outlined in SEQ ID NO: 1 in the sequence listing.

scFv-V2122 is prepared by binding a single-chain antibody (scFv) against CEACAM5 with the above-described V2122. This scFv-type anti-CEACAM5 antibody is an scFv sequence described in a patent document US7626011B2. The amino acid sequence of the scFv-type anti-CEACAM5 antibody is as outlined in SEQ ID NO: 2 in the sequence listing. In addition, the amino acid sequence of CEA-V2122 prepared by binding the scFv-type anti-CEACAM5 antibody with V2122 via an amino acid linker (GGGGSGGGG) (SEQ ID NO: 7) is as outlined in SEQ ID NO: 3 in the sequence listing.

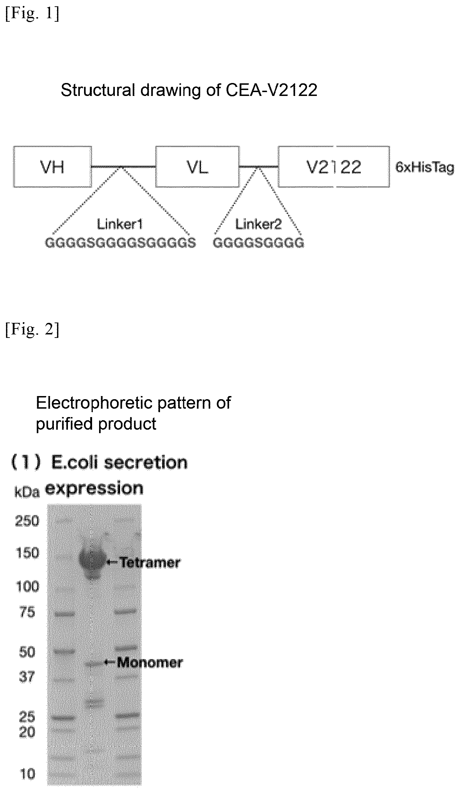

For the expression of a CEA-V2122 fusion protein, the DNA codon of a CEA-V2122 gene sequence, in which a pelB signal for secretion and expression in *Escherichia coli* had been incorporated into the N-terminus, and a 6xHis-Tag sequence had been incorporated into the C-terminus, was optimized for *Escherichia coli,* thereby synthesizing an artificial gene. This amino acid sequence is outlined in SEQ ID NO: 4 in the sequence listing, and the DNA sequence is summarized in SEQ ID NO: 5 in the sequence listing. Moreover, an outline of a domain structure is shown in Fig. 1.

A vector prepared by incorporating a chaperone skp gene into MCS2 of a pETDuet1 vector was used as a specific protein expression vector. Regarding the skp gene, the DNA codon was optimized for *Escherichia coli* based on the amino acid sequence outlined in SEQ ID NO: 6 in the sequence listing, thereby synthesizing an artificial gene. The synthesized skp gene was amplified by PCR using the primers (AAGGAGATATACATATGGATAAAATTGCCATTGTTAATAT (SEQ ID NO: 8), and TTGAGATCTGCCATATGTTATTTCACTTGTTTCAGAACG (SEQ ID NO: 9)), and the amplified gene was then cloned into MCS2 of the pETDue1 vector linearized with the restriction enzyme NdeI, using In-Fusion HD Cloning Kit, to obtain pETDuet_skp. Subsequently, the CEA-V2122 gene was incorporated into MCS1 of pETDuet_skp. Specifically, the artificially synthesized CEA-V2122 gene was amplified by PCR, using the primers (AGAAGGAGATATACCATGAAATATCTGCTGCCGAC (SEQ ID NO: 10), and CGCCGAGCTCGAATTTTAATGATGGTGATGATGATG (SEQ ID NO: 11)). Moreover, pETDuet_skp was linearized by PCR, using the primers (GGTATATCTCCTTCTTAAAGTTAAAC (SEQ ID NO: 12), and AATTCGAGCTCGGCGCGCCTGCAG (SEQ ID NO: 13)). The CEA-V2122 amplified by PCR and the linearized pETDuet_skp were subjected to cloning using In-Fusion HD Cloning Kit. The cloned vector was confirmed by sequencing in terms of the gene sequence incorporated therein, and after that, it was referred to as pETDuet_CEA-V2122_skp.

For the expression of the protein, pETDuet_CEA-V2122_skp was transformed into BL21(DE3) (Nippon Gene Co., Ltd.), which was then pre-cultured in a 2xYT medium (SIGMA-ALDRICH) at 37°C overnight. The medium used in the pre-culture was added to a new medium for 100-fold dilution, and the culture was then carried out at 37°C until OD (600 nm) became 0.5 to 2.0. Subsequently, IPTG was added to the culture to a final concentration of 0.5 mM, and the obtained mixture was then cultured at 37°C for 4 hours. After that, the culture supernatant was recovered and was then preserved at 4°C.

The CEA-V2122 protein was roughly purified according to a batch method utilizing 6xHis-Tag added to the C-terminus. Specifically, cOmplete His-Tag Purification Resin equilibrated with buffer A (50 mM Tris-HCl, 0.2 M NaCl, 1 mM EDTA, and 5 mM Imidazole; pH 8.0) was added to the culture supernatant preserved at 4°C. The obtained mixture was stirred for 2 hours to overnight at 4° C so that the protein was allowed to bind to the resin. Subsequently, the resin was recovered into a column, and a 20-column volume of washing operation was performed with buffer A. after that, a roughly purified product of CEA-V2122 was recovered by elution with buffer B (50 mM Tris-HCl, 0.2 M NaCl, 1 mM EDTA, and 400 mM Imidazole; pH 8.0).

Subsequently, the roughly purified product was purified using a Protein L column. Precisely, 1 mL of Capto L (GE Healthcare Life Sciences) was filled into a PD-10 column and equilibrated with 10 column volumes of PBS. That above roughly purified product was then applied to it. After that, the resultant was washed with 10 column volumes of PBS, eluted with 10 mM glycine hydrochloride (pH 2.0), and subjected to centrifugal concentration using Vivaspin Turbo 15 (MWCO 100,000). Moreover, using PD-10 (GE Healthcare Life Science), the buffer was replaced with PBS, and centrifugal concentration was further carried out using Vivaspin Turbo 4 (MWCO 100,000) to obtain a finally purified product. After completion of SDS-PAGE electrophoresis, the purity of tetramer CEA-V2122 was assayed by CBB staining. The results are shown in Fig. 2. As an SDS-PAGE gel, Mini-PROTEAN TGX 4-15% (Bio-Rad) was used, and as a CBB staining solution, Bullet CBB Stain One (Ready To Use) (Nacalai Tesque, Inc.) was used.

From Fig. 2, it was confirmed that the purified CEA-V2122 comprises an approximately 150 kDa tetramer as the main component.

### (2) Evaluation of the performance of CEA-V2122 by SPR

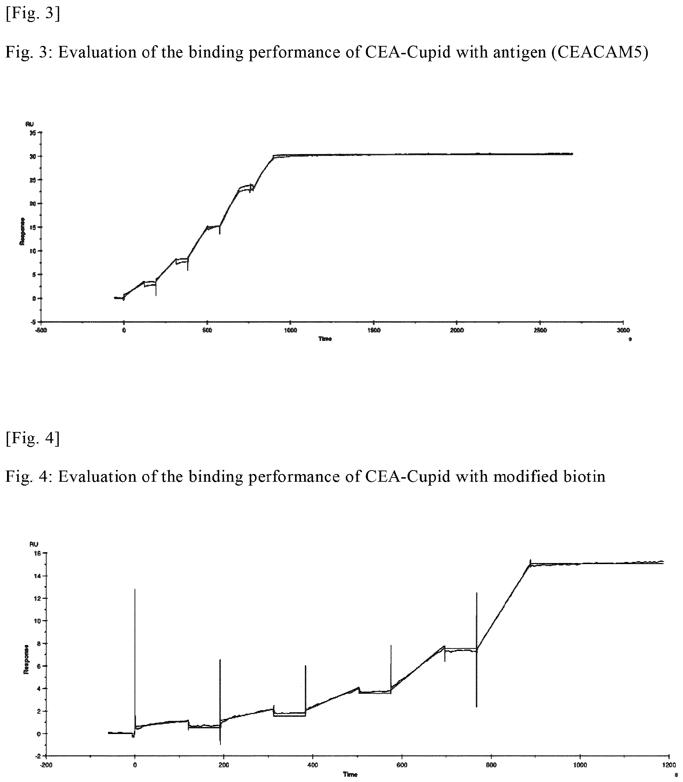

The affinity of CEA-V2122 for the antigen CEACAM5 was evaluated using a surface plasmon resonance (SPR) measuring device, Biacore T200 (GE Healthcare Life Sciences). Specifically, Recombinant Human CEACAM-5/CD66e Protein, CF (R & D SYSTEMS) was immobilized on Sensor Chip CM5 (GE Healthcare Life Sciences) using an amine-coupling kit (GE Healthcare Life Sciences). The final amount of the ligand immobilized was 279 RU. Moreover, about the purified CEA-V2122, two-fold serial dilutions from 1E-08 M to 6.25E-10 M were prepared as analytes. Regarding interaction analysis, data were obtained by single-cycle kinetics analysis. Using Biacore T200 Evaluation Software, version 2.0, the obtained data were subjected to curve fitting in a bivalent analysis mode, and the following values were obtained: ka1 = 3.208E + 5, and kd1 = 3.461E - 7. Moreover, since evaluation can be carried out at K_{D} = kd1/ka1 in the bivalent analysis, the evaluation value K_{D} = kd1/ka1 = 3.461E - 7 / 3.208E + 5 = 1.078E - 12 was obtained. These results are shown in Fig. 3.

The K_{D} value in the sensorgram shown in Fig. 3 confirmed that CEA-V2122 strongly binds to CEACAM5.

Furthermore, the interaction between CEA-V2122 and modified biotin was also analyzed using Biacore T200. The modified biotin was the title compound 14 described in Example 1 of International Publication WO2018/07239. In addition, the analysis method was specifically as follows. An amine-coupling kit was used, a target value was set to be 5000 RU, and the purified CEA-V2122 was immobilized on Sensor Chip CM5. Regarding the concentrations of the analytes, five types of two-fold serial dilutions from 1E-08 M to 6.25E-10 M were used. Regarding interaction analysis, data were obtained by single-cycle kinetics analysis. Using Biacore T200 Evaluation Software, version 2.0, the obtained data were subjected to curve fitting in a bivalent analysis mode, and the following values were obtained: ka1 = 3.792E + 4, and kd1 = 4.424E - 6. Moreover, since evaluation can be carried out at K_{D} = kd1/ka1 in the bivalent analysis, the evaluation value K_{D} = kd1/ka1 = 3.792E + 4/4.424E - 6 = 1.167E - 10 was obtained. These results are shown in Fig. 4.

The K_{D} value in the sensorgram shown in Fig. 4 confirmed that CEA-V2122 strongly binds to modified biotin.

### < Cupid labeled with ¹²⁵I >

Cupid labeled with ¹²⁵I was produced by the following method.

11 µL of a Methanol solution containing N-Chlorosuccinimide (1mg/mL), 12 µL of Na¹²⁵I solution (23.2 MBq), and 41 µL of 1% acetic acid/methanol were added to a reaction vessel, in which 38.2 µg of *N*-Succinimidyl 3-Trimethylstannyl-benzoate (abbreviation: ATE, Toronto Research Chemicals, North York, Canada) had previously been placed, and the obtained mixture was then stirred at room temperature for 45 minutes.

40 µL of Ultrapure water was added and mixed with the reaction solution. The thus obtained mixture was purified by reverse phase HPLC (mobile phase A: 1% CF₃COOH/water, mobile phase B: 1% CF₃COOH/MeCN; mobile phase B: 40%-60% (20 minutes), retention time: 8.7 min, column: Cosmosil 5C₁₈-AR-II (4.6 x 15 mm, Nacalai Tesque, Kyoto, Japan), flow rate: 1.0 mL/min) to collect a fraction comprising [¹²⁵I]SIB, thereby obtaining N-Succinimidyl 3-[¹²⁵I]iodobenzoate ([¹²⁵I]SIB). The solvent was evaporated to dry from the [125I]SIB collection solution recovered by the reverse phase HPLC. The residue was then re-dissolved in 25 µL of ultrapure water.

An scFv-CEA-Cupid solution [4.1 mg/mL] (50 µL) and a 0.1 M borate buffer (pH 8.5) (25 µL) were added to 25 µL of the [¹²⁵I]SIB aqueous solution, and the obtained mixture was then reacted under ice cooling conditions for 2 hours. Afterward, the reaction solution was passed through a PD-10 column (GE Healthcare, Uppsala, Sweden) and was eluted with PBS to obtain compound X. The separated sample was assayed by thin-layer chromatography using Tec-control Chromatography 150-771 strip (BIODEX, Shirley, NY, USA) and size exclusion HPLC.

- Labeling percentage upon SIB synthesis
   75.6%(SIB that could be recovered as a fraction from HPLC)
   * 92.6% on HPLC
- ¹²⁵I-Cupid labeling percentage
   49.4%(¹²⁵I-Cupid removed from PD-10)
   * 66.1% on SE-HPLC
- Radiochemical purity
   SE-HPLC: 98.0%
   iTLC: 99.7%

### References:

Zalutsky MR, Narula AS. Astatination of proteins using an N-succinimidyl tri-n-butylstannyl benzoate intermediate. Int J Rad Appl Instrum A. 1988; 39(3): 227-32.
[Experiment 1: Studies regarding the pharmacokinetics of ¹²⁵I-Cupid-scFv antibody in MKN-45 cancer-bearing mouse models transplanted with stomach cancer cells (MKN-45) highly expressing carcinoembryonic antigen (CEA)]

### < Purpose >

Before evaluating the pharmacokinetics of ²¹¹At-psyche against a Cupid-scFv antibody binding to CEA that is expressed in cancer cells, the binding rate of the Cupid-scFv antibody to the cancer cells, clearance from the blood, and the rate of accumulation in normal tissues need to be grasped. Hence, in the present studies, the pharmacokinetics of the Cupid-scFv antibody in MKN-45 cancer-bearing mouse models were examined.

### < Method >

The present experiment used highly CEA-expressing human stomach cancer cells (MKN-45). 9-week-old male immunodeficient mice (BALB/cSlc nu/nu) were used. MKN-45 cells (1 x 10⁷/0.1 ml) were transplanted into the mice's subcutis on the right shoulder dorsal side to produce MKN-45 cancer-bearing mouse models. The mice were divided into eight groups at the time point in which the tumor volume became a predetermined size (on Day 13 after the transplantation), and a ¹²⁵I-labeled anti-Cupid-scFv antibody (¹²⁵I-Cupid-scFv antibody) was administered in an amount of 100 pmol (about 0.18 MBq/100 ml) via the caudal vein of each mouse. In addition, to evaluate the binding amount of the Cupid-scFv antibody to CEA depending on the amount of the Cupid-scFv antibody administered, the Cupid-scFv antibody was administered in an amount of 50 pmol to one group. One minute, 1 hour, 6 hours, 12 hours, 24 hours (100 pmol), 24 hours (50 pmol), 48 hours, and 72 hours after administration of the ¹²⁵I-Cupid-scFv antibody, the mice were euthanized by collecting whole blood from the heart of each mouse under isoflurane anesthesia. Tumor tissues and normal tissues/organs were then excised. Normal tissues/organs: skin (lower right limb), muscle (right gastrocnemius muscle), heart, lung, spleen, pancreas, white fat (upper portion of right side testis), testis (right side), stomach (not including contents), small intestine (including contents), large intestine (including contents), liver, brown fat (interscapular), submandibular gland, thyroid gland (together with trachea), bone (right femur), cerebrum, eyeballs (both sides) and bladder (not including urine); the remaining residues were divided into three equal parts, and the three equal parts were defined to be residue-1, residue-2, and residue-3. After that, the weights of blood, plasma, tumor, and normal tissues/organs were measured, and the radioactivity thereof was then measured using a γ counter. The amounts of the ¹²⁵I-Cupid-scFv antibody (radioactivity) accumulated in the blood, plasma, tumor, and normal tissues/organs were calculated. That is to say, the rates (% ID/g) of the ¹²⁵I-Cupid-scFv antibody accumulated per unit weight (g) of the blood, plasma, tumor, and normal tissues/organs, in the administered ¹²⁵I-Cupid-scFv antibody were shown. Regarding accumulation of the ¹²⁵I-Cupid-scFv antibody in the thyroid gland, since the weight of the thyroid gland could not be precisely measured, it was shown that the rate (% ID) of the ¹²⁵I-Cupid-scFv antibody accumulated in the thyroid gland in the administered ¹²⁵I-Cupid-scFv antibody.

### < Results >

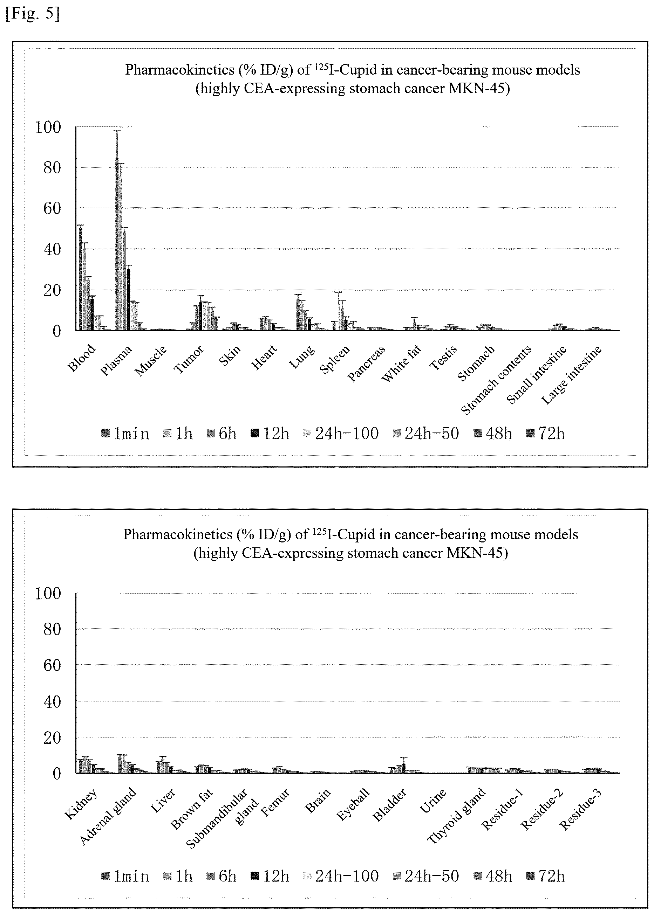

Fig. 5 shows the biodistribution (% ID/g) of the ¹²⁵I-Cupid-scFv antibody.

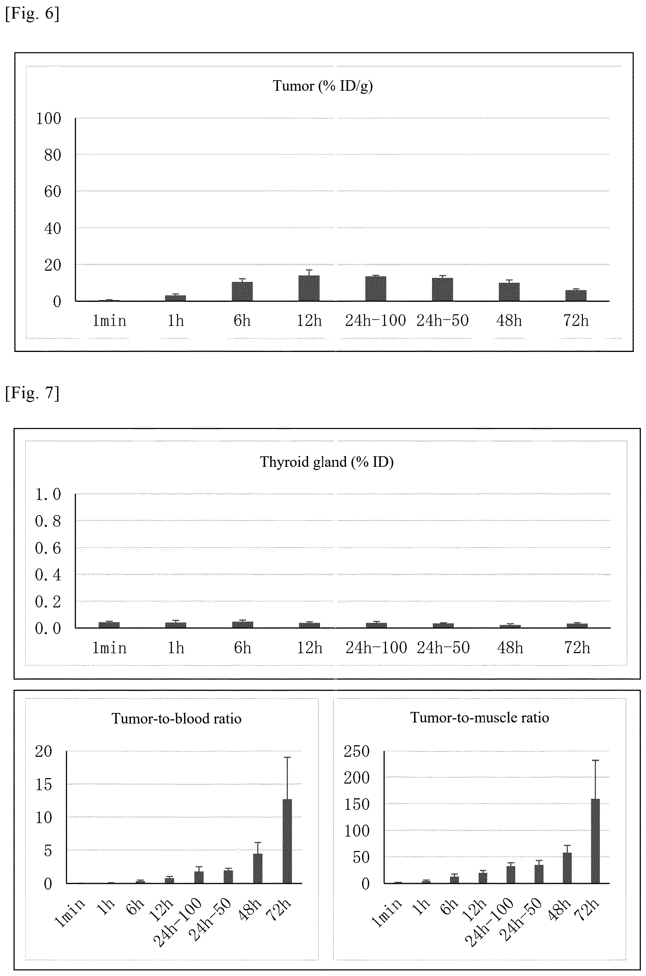

Fig. 6 shows the amount (% ID/g) of the ¹²⁵I-Cupid-scFv antibody accumulated in tumor tissues.
Fig. 7 shows the amount (% ID) of the ¹²⁵I-Cupid-scFv antibody accumulated in the thyroid gland, the tumor-to-blood ratio, and the tumor-to-muscle ratio.

The Clearance of the ¹²⁵I-Cupid-scFv antibody from the blood was 50.0±1.6% after 1 minute, 40.1±2.8% after 1 hour, 25.1±1.4% ID/g after 6 hours, 15.5±1.4% ID/g after 12 hours, 6.4±0.8% ID/g (50 pmol; 6.6±0.5% ID/g) after 24 hours, 1.9±0.2% ID/g after 48 hours, and 0.4±0.1% ID/g after 72 hours. That is, the excretion of the ¹²⁵I-Cupid-scFv antibody from the blood decreased as time passed, to about 94% of the administered amount after 24 hours, about 98% after 48 hours, and about 99.6% after 72 hours.

Accumulation of the ¹²⁵I-Cupid-scFv antibody in the tumor was 0.6±0.2% ID/g after 1 minute, 3.2±0.6% ID/g after 1 hour, 10. 4±1.7% ID/g after 6 hours, 14.0±3.1% ID/g after 12 hours, 13.5±0.6% (50 pmol; 12.6±1.3% ID/g) after 24 hours, 9.9±1.6% ID/g after 48 hours, and 5.9±0.7% ID/g after 72 hours. The accumulation of the ¹²⁵I. Cupid-scFv antibody in the tumor increased as time passed and peaked after 12 hours. High accumulation was maintained after 12 hours and 14 hours. After 72 hours, the accumulation of the ¹²⁵I-Cupid-scFv antibody in the tumor was decreased to about half of the peak. Accumulating the 125I-Cupid-scFv antibody in the tumor was almost the same between the doses of 100 pmol and 50 pmol 24 hours after the administration.

The tumor-to-blood ratio was 0.01±0.00 after 1 minute, 0.06±0.03 after 1 hour, 0.33±0.16 after 6 hours, 0.79±0.28 after 12 hours, 1.79±0.71 (50 pmol; 1.94±0.33) after 24 hours, 4.43±1.75 after 48 hours, and 12.74±6.24 after 72 hours.

The tumor-to-muscle ratio was 1.5±0.3 after 1 minute, 4.0±2.2 after 1 hour, 12.7±5.0 after 6 hours, 19.7±4.4 after 12 hours, 32.1±6.5 (34.6±8.5) after 24 hours, 57.7±14.4 after 48 hours, and 159.8±72.0 after 72 hours.

Accumulation of the ¹²⁵I-Cupid-scFv antibody in the thyroid gland was considerably low (less than 0.05% ID). In addition, an increasing tendency was not observed as time passed.

### [Experiment 2: Studies regarding the pharmacokinetics of Cupid-scFv antibody (24h before) + ²¹¹At-psyche-B in MKN-45 cancer-bearing mouse models]

### < Purpose >

The pharmacokinetics of ²¹¹At-psyche-B against the Cupid-scFv antibody that had previously been administered (24h before) to MKN-45 cancer-bearing mouse models were examined.

### < Method >

The present experiment used highly CEA-expressing human stomach cancer cells (MKN-45). 9-week-old male immunodeficient mice (BALB/cSlc nu/nu) were used. MKN-45 cells (1 x 10⁷/0.1 ml) were transplanted into the mice's subcutis on the right shoulder dorsal side to produce MKN-45 cancer-bearing mouse models. The mice were divided into four groups when the tumor volume became a predetermined size (on Day 13 after the transplantation). A Cupid-scFv antibody was administered in 100 pmol/0.1 ml via the caudal vein of each mouse. Twenty-four hours after administration of the Cupid-scFv antibody, ²¹¹At-psyche-B (about 0.25 MBq/100 ml) was administered via the caudal vein of the mice. One minute, 1 hour, 6 hours, and 24 hours after administration of the ²¹¹At-psyche-B, the mice were euthanized by collecting whole blood from the heart of each mouse under isoflurane anesthesia, and tumor tissues and normal tissues/organs were then excised. Normal tissues/organs: skin (lower right limb), muscle (right gastrocnemius muscle), heart, lung, spleen, pancreas, white fat (upper portion of right side testis), testis (right side), stomach (without contents), small intestine, large intestine, liver, brown fat, submandibular gland, thyroid gland (together with trachea), bone (right femur), cerebrum, eyeballs (both sides), bladder, and urine; the remaining residues were divided into three equal parts, and the three equal parts were defined to be residue-1, residue-2, and residue-3. After that, the weights of blood, plasma, urine, tumor, and normal tissues/organs were measured, and the radioactivity thereof was then measured using a γ counter. The amounts of the ²¹¹At-psyche-B (radioactivity) accumulated in the blood, plasma, urine, tumor, and normal tissues/organs were calculated. That is to say, the rates (% ID/g) of the ²¹¹At-psyche-B accumulated per unit weight (g) of the blood, plasma, urine, tumor, and normal tissues/organs, in the administered ²¹¹At-psyche-B, were shown. Regarding accumulation of the ²¹¹At-psyche-B in the thyroid gland, since the weight of the thyroid gland could not be precisely measured, it was shown that the rate (% ID) of the ²¹¹At-psyche-B accumulated in the thyroid gland in the administered ²¹¹At-psyche-B.

### < Results >

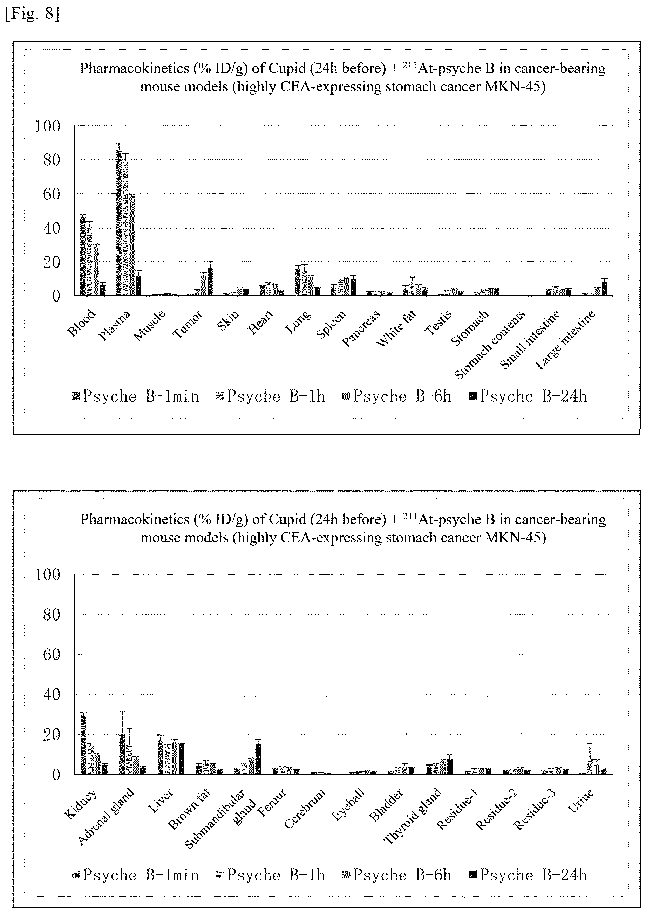

Fig. 8 shows the biodistribution (% ID/g) of the Cupid-scFv antibody (24h before) + ²¹¹At-psyche-B.

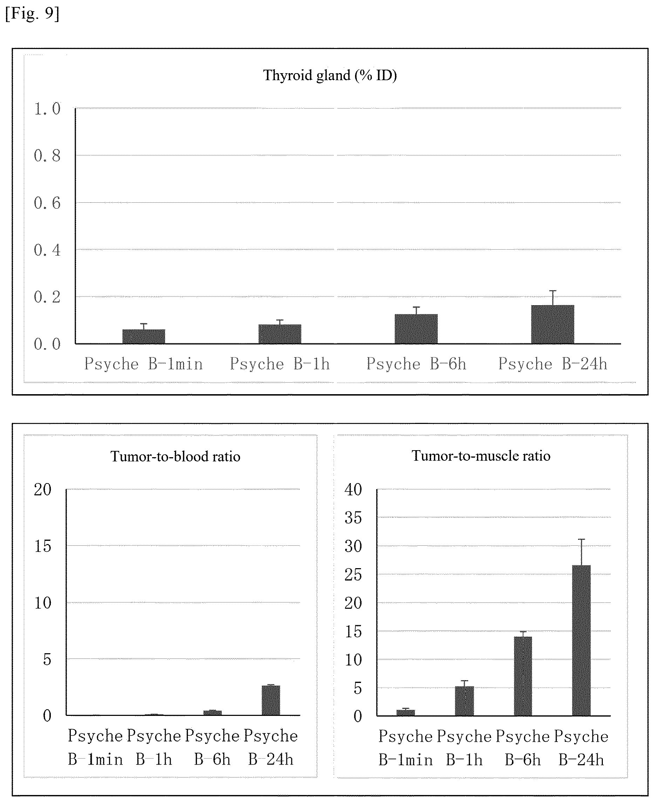

Fig. 9 shows the accumulation (% ID) of the Cupid-scFv antibody (24h before) + ²¹¹At-psyche-B in the thyroid gland, the tumor-to-blood ratio, and the tumor-to-muscle ratio.

The Clearance of the ²¹¹At-psyche-B from the blood was 46.4±1.4% ID/g after 1 minute, 40.6±3.2% ID/g after 1 hour, 29.4±0.9% ID/g after 6 hours, and 6.2±1.3% ID/g after 24 hours. The accumulation of the ²¹¹At-psyche-B in the blood decreased as time passed, and 94% of the administered amount was excreted after 24 hours.

Accumulation of the ²¹¹At-psyche-B in the tumor was 0.6±0.1% ID/g after 1 minute, 3.2±0.4% ID/g after 1 hour, 11.8±1.5% ID/g after 6 hours, and 16.3±4.1% after 24 hours. That is, the accumulation of the ²¹¹At-psyche-B in the tumor increased as time passed.

The tumor-to-blood ratio was 0.01±0.00 after 1 minute, 0.08±0.01 after 1 hour, 0.40±0.05 after 6 hours, and 2.63±0.10 after 24 hours.

The tumor-to-muscle ratio was 1.5±0.3 after 1 minute, 5.2±1.0 after 1 hour, 14.0±0.9 after 6 hours, and 26.5±4.6 after 24 hours.

Accumulation of the ²¹¹At-psyche-B in the thyroid gland was low (0.16±0.06% ID after 24h), but an increasing tendency was observed as time passed.

Accumulation of the ²¹¹At-psyche-B in the liver was 17.4±2.3% ID/g after 1 minute, 13.6±1.4% ID/g after 1 hour, 16.0±1.4% ID/g after 6 hours, and 15.5±0.1% after 24 hours. That is, accumulation of the ²¹¹At-psyche-B in the liver was high immediately after administration thereof, and such a high value was maintained until 24 hours after the administration.

Accumulation of the ²¹¹At-psyche-B in the spleen was 4.9±1.7% ID/g after 1 minute, 7.9±1.1% ID/g after 1 hour, 10.1±0.3% ID/g after 6 hours, and 9.6±2.4% after 24 hours. That is, the accumulation of the ²¹¹At-psyche-B in the spleen increased as time passed, and it peaked 6 hours after the administration. Such a high value was maintained even 24 hours after the administration.

Accumulation of the ²¹¹At-psyche-B in the kidney was 29.3±1.5% ID/g after 1 minute, 14.2±1.3% ID/g after 1 hour, 9.8±0.7% ID/g after 6 hours, and 4.7±0.8% after 24 hours. That is, the accumulation of the ²¹¹At-psyche-B in the kidney was decreased as time passed, and after 24 hours, it was decreased to 5% of the administered amount.

Accumulation of the ²¹¹At-psyche-B in the lung was 15.9±1.7% ID/g after 1 minute, 14.7±3.6% ID/g after 1 hour, 11.2±0.9% ID/g after 6 hours, and 4.2±0.5% after 24 hours. That is, accumulation of the ²¹¹At-psyche-B in the lung was high until 6 hours after the administration, but after 24 hours, it was decreased to 4% of the administered amount.

### [Experiment 3: Studies regarding the pharmacokinetics of Cupid-scFv antibody (48h before) + ²¹¹At-psyche-B in MKN-45 cancer-bearing mouse models]

From the results of the experiment regarding the pharmacokinetics of the ¹²⁵I-Cupid-scFv antibody in the above Experiment 1, accumulation of the ¹²⁵I-Cupid-scFv antibody in the blood was low at 48 hours after administration of the ¹²⁵I-Cupid-scFv antibody. Still, the accumulation of the ¹²⁵I-Cupid-scFv antibody in the tumor exhibited a high value.

### < Purpose >

The pharmacokinetics of ²¹¹At-psyche-B against the Cupid-scFv antibody that had previously been administered (48h before) to the MKN-45 cancer-bearing mouse models were examined.

### < Method >

The present experiment highly used CEA-expressing human stomach cancer cells (MKN-45). 9-week-old male immunodeficient mice (BALB/cSlc nu/nu) were used. MKN-45 cells (1 x 10⁷/0.1 ml) were transplanted into the mice's subcutis on the right shoulder dorsal side to produce cancer-bearing mouse models. The mice were divided into four groups when the tumor volume became a predetermined size (on Day 12 after the transplantation). A Cupid-scFv antibody was administered in 100 pmol/0.1 ml via the caudal vein of each mouse. Forty-eight hours after administration of the Cupid-scFv antibody, ²¹¹At-psyche-B (about 0.25 MBq/100 ml) was administered via the caudal vein of the mice. One minute, 1 hour, 6 hours, and 24 hours after administration of the ²¹¹At-psyche-B, the mice were euthanized by collecting whole blood from the heart of each mouse under isoflurane anesthesia, and tumor tissues and normal tissues/organs were then excised. Normal tissues/organs: skin (lower right limb), muscle (right gastrocnemius muscle), heart, lung, spleen, pancreas, white fat (upper portion of right side testis), testis (both sides), stomach, stomach contents, small intestine (including contents), large intestine (including contents), liver, brown fat (interscapular), submandibular gland, thyroid gland (together with trachea), bone (right femur), cerebrum, eyeballs (both sides), bladder, and urine; the remaining residues were divided into three equal parts, and the three equal parts were defined to be residue-1, residue-2, and residue-3. After that, the weights of blood, plasma, urine, tumor, and normal tissues/organs were measured, and the radioactivity thereof was then measured using a γ counter. The amounts of the ²¹¹At-psyche-B (radioactivity) accumulated in the blood, plasma, urine, tumor, and normal tissues/organs were calculated. That is to say, the rates (% ID/g) of the ²¹¹At-psyche-B accumulated per unit weight (g) of the blood, plasma, urine, tumor, and normal tissues/organs, in the administered ²¹¹At-psyche-B, were shown. Regarding accumulation of the ²¹¹At-psyche-B in the thyroid gland, since the weight of the thyroid gland could not be precisely measured, it was shown that the rate (% ID) of the ²¹¹At-psyche-B accumulated in the thyroid gland in the administered ²¹¹At-psyche-B.

### < Results >

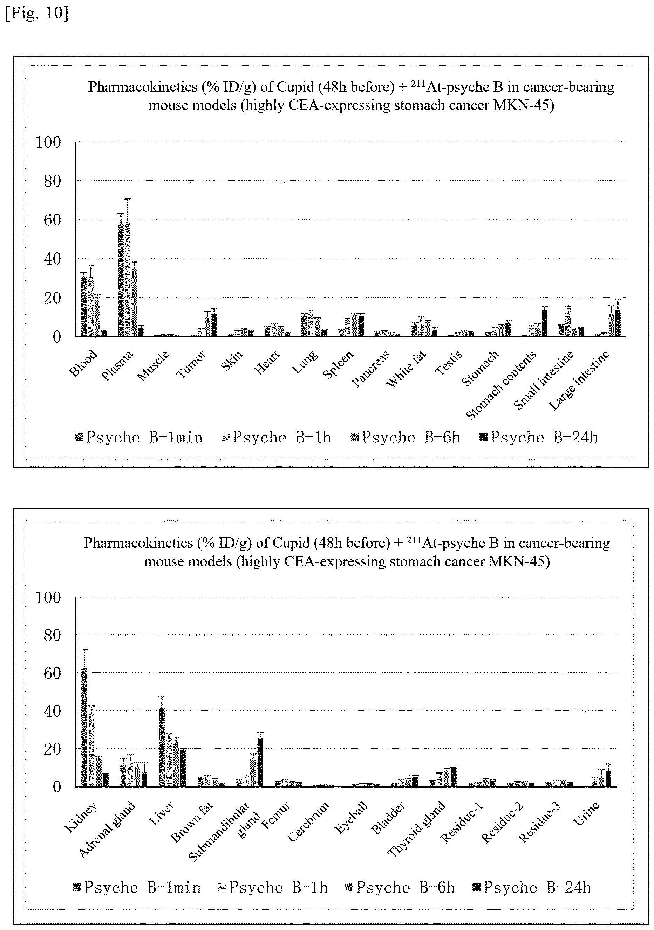

Fig. 10 shows the biodistribution (% ID/g) of the Cupid-scFv antibody (48h before) + ²¹¹At-psyche-B.

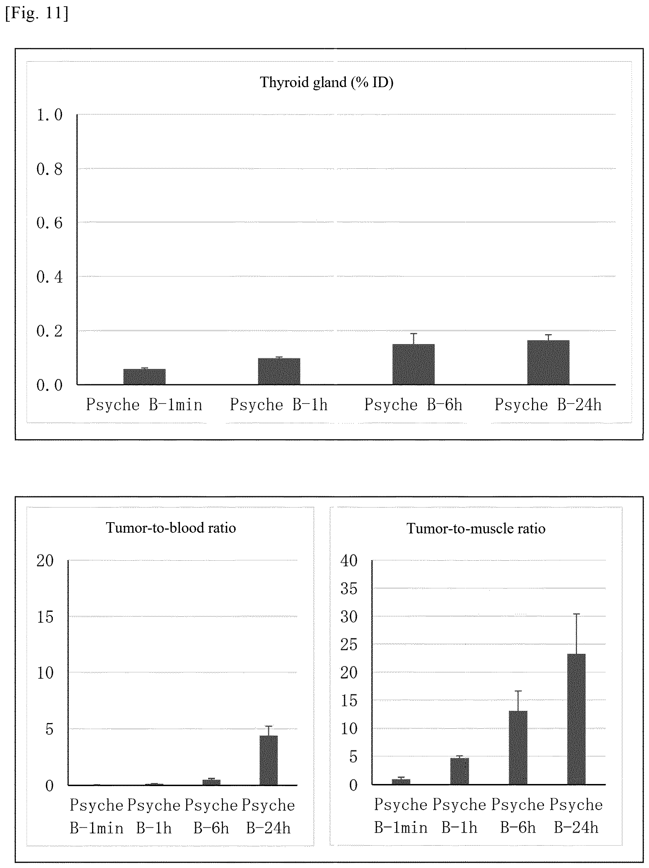

Fig. 11 shows the amount (% ID) of the Cupid-scFv antibody (48h before) + ²¹¹At-psyche-B in the thyroid gland, the tumor-to-blood ratio, and the tumor-to-muscle ratio.

The Clearance of the ²¹¹At-psyche-B from the blood was 30.7±2.2% ID/g after 1 minute, 30.8±5.5% ID/g after 1 hour, 19.0±2.6% ID/g after 6 hours, and 2.6±0.5% ID/g after 24 hours. That is, the accumulation of the ²¹¹At-psyche-B in the blood was the same level at 1 minute and 1 hour after the administration, and after 24 hours, it was decreased to about 2.6% of the administered amount.

Accumulation of the ²¹¹At-psyche-B in the tumor was 0.5±0.1% ID/g after 1 minute, 3.2±0.7% ID/g after 1 hour, 9.9±2.9% ID/g after 6 hours, and 11.3±3.3% after 24 hours. That is, the accumulation of the ²¹¹At-psyche-B in the tumor increased as time passed.

The tumor-to-blood ratio was 0.02±0.00 after 1 minute, 0.11±0.02 after 1 hour, 0.51±0.09 after 6 hours, and 4.38±0.85 after 24 hours.

The tumor-to-muscle ratio was 1.0±0.3 after 1 minute, 4.7±0.4 after 1 hour, 13.1±3.5 after 6 hours, and 23.2±7.2 after 24 hours.

Accumulation of the ²¹¹At-psyche-B in the thyroid gland was low (0.16±0.02% ID after 24h), but an increasing tendency was observed as time passed.

Accumulation of the ²¹¹At-psyche-B in the liver was 41.4±6.2% ID/g after 1 minute, 25.6±2.5% ID/g after 1 hour, 23.6±2.3% ID/g after 6 hours, and 19.3±0.6% after 24 hours. That is, the ²¹¹At-psyche-B in the liver was high immediately after administration. Although it decreased as time passed, such a high value was maintained 24 hours after the administration.

Accumulation of the ²¹¹At-psyche-B in the spleen was 3.5±0.1% ID/g after 1 minute, 8.5±0.6% ID/g after 1 hour, 11.1±0.7% ID/g after 6 hours, and 10.2±1.6% after 24 hours. That is, the accumulation of the ²¹¹At-psyche-B in the spleen increased as time passed, and it peaked 6 hours after the administration. Such a high value was maintained even 24 hours after the administration.

Accumulation of the ²¹¹At-psyche-B in the kidney was 62.3±9.8% ID/g after 1 minute, 38.0±4.5% ID/g after 1 hour, 14.9±0.8% ID/g after 6 hours, and 6.6±0.2% after 24 hours. That is, the ²¹¹At-psyche-B was excreted from the kidney immediately after administration thereof, and after 24 hours, the amount of ²¹¹At-psyche-B was decreased to 6.6% of the administered amount.

Accumulation of the ²¹¹At-psyche-B in the lung was 10.2±1.6% ID/g after 1 minute, 12.2±0.9% ID/g after 1 hour, 8.5±0.9% ID/g after 6 hours, and 3.5±0.2% after 24 hours. That is, the accumulation of the ²¹¹At-psyche-B in the lung reached a peak 1 hour after the administration, and after that, the amount of the ²¹¹At-psyche-B accumulated in the lung decreased as time passed. After 24 hours, it decreased to 3.5% of the administered amount.

### [Experiment 4: Studies regarding the pharmacokinetics of ²¹¹At-psyche-B in MKN-45 cancer-bearing mouse models]

### < Purpose >

The pharmacokinetics of ²¹¹At-psyche-B was examined in the absence of the Cupid-scFv antibody in MKN-45 cancer-bearing mouse models.

### < Method >

The present experiment used human stomach cancer cells (MKN-45) expressing carcinoembryonic antigen (CEA). 9-week-old male immunodeficient mice (BALB/cSlc nu/nu) were used. MKN-45 cells (1 x 10⁷/0.1 ml) were transplanted into the mice's subcutis on the right shoulder dorsal side to produce cancer-bearing mouse models. The mice were divided into four groups when the tumor volume became a predetermined size (on Day 14 after the transplantation), and ²¹¹At-psyche-B (about 0.25 MBq/00 ml) was administered via the caudal vein of each mouse. One minute, 1 hour, 6 hours, and 24 hours after administration of the ²¹¹At-psyche-B, the mice were euthanized by collecting whole blood from the heart of each mouse under isoflurane anesthesia, and tumor tissues and normal tissues/organs were then excised. Normal tissues/organs: skin (lower right limb), muscle (right gastrocnemius), heart, lung, spleen, pancreas, white fat (upper portion of right side testis), testis (both sides), stomach, stomach contents, small intestine (including contents), large intestine (including contents), liver, brown fat (interscapular), submandibular gland, thyroid gland (together with trachea), bone (right femur), brain, eyeball, bladder, and urine; the remaining residues were divided into three equal parts, and the three equal parts were defined to be residue-1, residue-2, and residue-3. After that, the weights of blood, plasma, urine, tumor, and normal tissues/organs were measured, and the radioactivity thereof was then measured using a γ counter. The amounts of the ²¹¹At-psyche-B (radioactivity) accumulated in the blood, plasma, urine, tumor, and normal tissues/organs were calculated. That is to say, the rates (% ID/g) of the ²¹¹At-psyche-B accumulated per unit weight (g) of the blood, plasma, urine, tumor, and normal tissues/organs, in the administered ²¹¹At-psyche-B, were shown. Regarding accumulation of the ²¹¹At-psyche-B in the thyroid gland, since the weight of the thyroid gland could not be precisely measured, it was shown that the rate (% ID) of the ²¹¹At-psyche-B accumulated in the thyroid gland in the administered ²¹¹At-psyche-B.

### < Results >

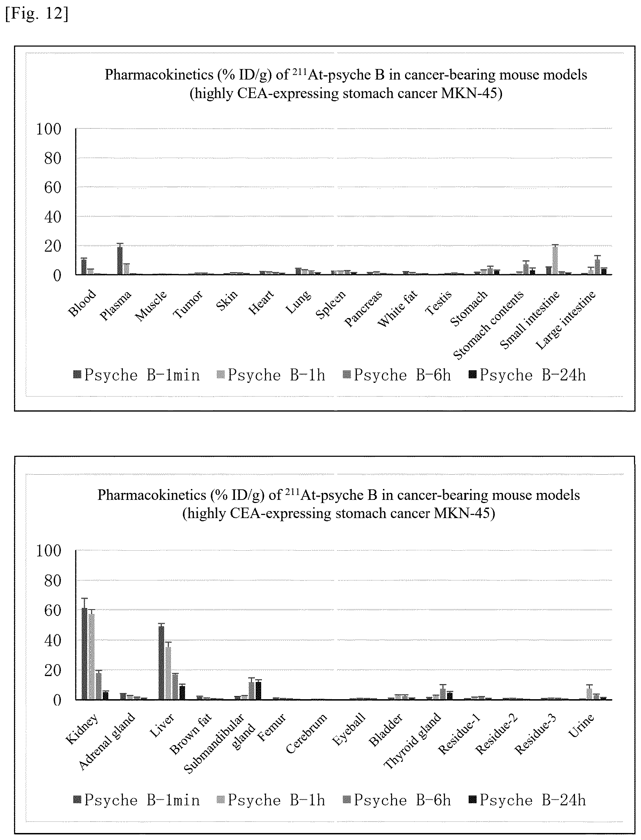

Fig. 12 shows the biodistribution (% ID/g) of ²¹¹At-psyche-B.

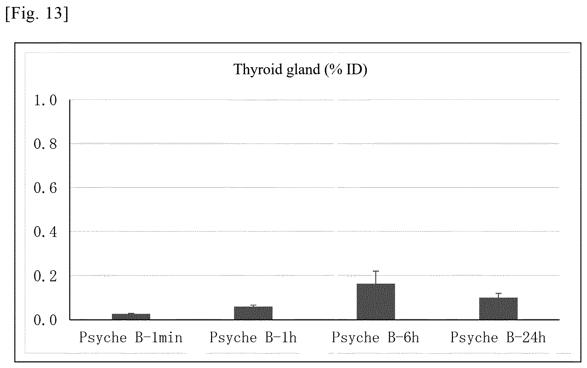

Fig. 13 shows the amount (% ID) of ²¹¹At-psyche-B in the thyroid gland.

Accumulation of the ²¹¹At-psyche-B in the blood was 10.4±1.1% ID/g after 1 minute, 3.8±0.3% ID/g after 1 hour, 0.4±0.1% ID/g after 6 hours, and 0.2±0.04% ID/g after 24 hours. That is, the accumulation of the ²¹¹At-psyche-B in the blood was already low at 1 minute, and after 24 hours, it was decreased to about 0.2% of the administered amount. Thus, clearance from the blood was considerably rapid.

Accumulation of the ²¹¹At-psyche-B in the tumor was 0.3±0.05% ID/g after 1 minute, 1.0±0.1% ID/g after 1 hour, 1.0±0.1% ID/g after 6 hours, and 0.3±0.02% after 24 hours. That is, accumulation of the ²¹¹At-psyche-B in the tumor was hardly observed.

Accumulation of the ²¹¹At-psyche-B in the thyroid gland was low; after 6 hours, it was 0.16±0.06% ID.

Accumulation of the ²¹¹At-psyche-B in the kidney was 61.4±6.5% ID/g after 1 minute, 57.2±3.1% ID/g after 1 hour, 17.7±1.9% ID/g after 6 hours, and 5.1±0.9% after 24 hours. That is, the ²¹¹At-psyche-B was accumulated in the kidney immediately after administration, but after 24 hours, the amount of ²¹¹At-psyche-B accumulated in the kidney was decreased to 5.1% of the administered amount.

Accumulation of the ²¹¹At-psyche-B in the liver was 49.1±6.2% ID/g after 1 minute, 35.3±3.3% ID/g after 1 hour, 16.8±0.7% ID/g after 6 hours, and 9.0±1.4% after 24 hours. That is, the ²¹¹At-psyche-B in the liver was high immediately after administration. Although it decreased as time passed, such a high value was maintained 24 hours after the administration.

### [Experiment 5: Studies regarding the pharmacokinetics of Cupid-scFv antibody (24h before) + ²¹¹At-psyche-BR in MKN-45 cancer-bearing mouse models]

### < Purpose >

The pharmacokinetics of ²¹¹At-psyche-BR to the Cupid-scFv antibody that had previously been administered (24h before) to MKN-45 cancer-bearing mouse models were examined.

### < Method >

The present experiment used human stomach cancer cells (MKN-45) expressing carcinoembryonic antigen (CEA). 9-week-old male immunodeficient mice (BALB/cSlc nu/nu) were used. MKN-45 cells (1 x 10⁷/0.1 ml) were transplanted into the mice's subcutis on the right shoulder dorsal side to produce cancer-bearing mouse models. The mice were divided into four groups when the tumor volume became a predetermined size (on Day 13 after the transplantation). The Cupid-scFv antibody was administered in 100 pmol/0.1 ml via the caudal vein of each mouse. Twenty-four hours after administration of the Cupid-scFv antibody, ²¹¹At-psyche-BR (about 0.25 MBq/100 ml) was administered via the caudal vein of each mouse. One minute, 1 hour, 6 hours, and 24 hours after administration of the ²¹¹At-psyche-BR, the mice were euthanized by collecting whole blood from the heart of each mouse under isoflurane anesthesia, and tumor tissues and normal tissues/organs were then excised. Normal tissues/organs: skin (lower right limb), muscle (right gastrocnemius muscle), heart, lung, spleen, pancreas, white fat (upper portion of right side testis), testis (both sides), stomach, stomach contents, small intestine (including contents), large intestine (including contents), liver, brown fat (interscapular), submandibular gland, thyroid gland (together with trachea), bone (right femur), cerebrum, eyeballs (both sides), bladder, and urine; the remaining residues were divided into three equal parts, and the three equal parts were defined to be residue-1, residue-2, and residue-3. After that, the weights of blood, plasma, urine, tumor, and normal tissues/organs were measured, and the radioactivity thereof was then measured using a γ counter. The amounts of the ²¹¹At-psyche-BR (radioactivity) accumulated in the blood, plasma, urine, tumor, and normal tissues/organs were calculated. That is to say, the rates (% ID/g) of the ²¹¹At-psyche-BR accumulated per unit weight (g) of the blood, plasma, urine, tumor, and normal tissues/organs, in the administered ²¹¹At-psyche-BR were shown. Regarding accumulation of the ²¹¹At-psyche-BR in the thyroid gland, since the weight of the thyroid gland could not be precisely measured, it was shown that the rate (% ID) of the ²¹¹At-psyche-BR accumulated in the thyroid gland in the administered ²¹¹At-psyche-BR.

### < Results >

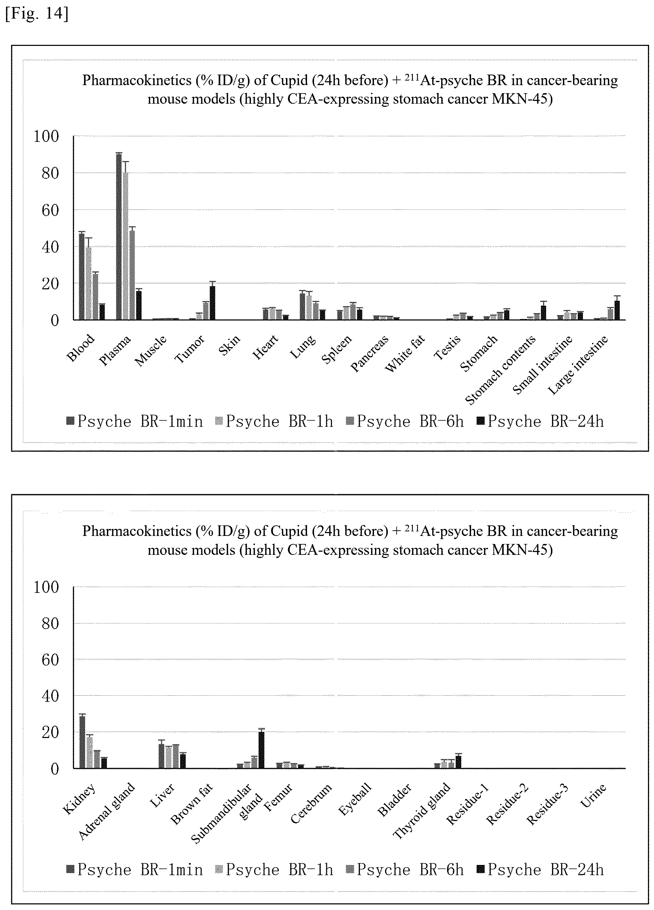

Fig. 14 shows the biodistribution (% ID/g) of the Cupid-scFv antibody (24h before) + ²¹¹At-psyche-BR.

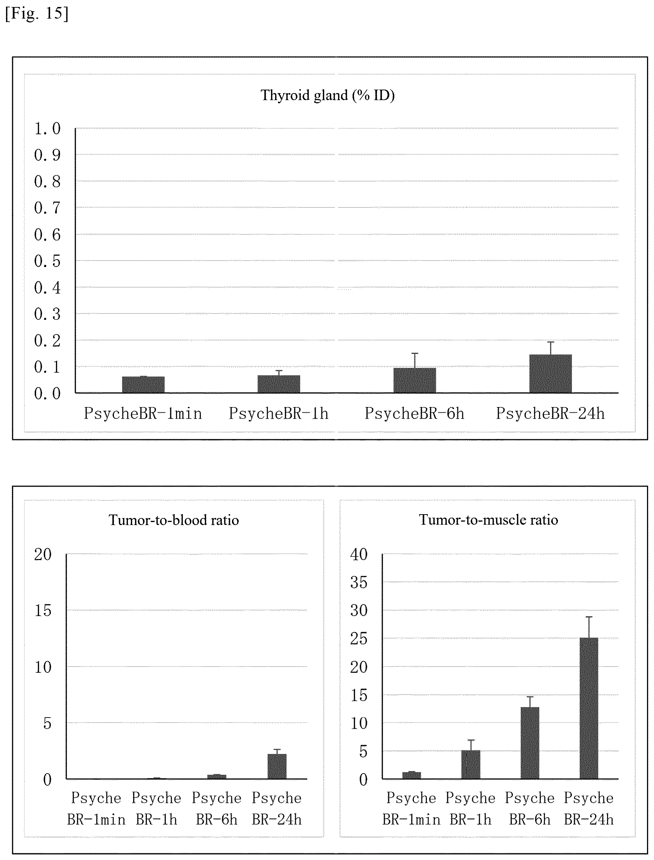

Fig. 15 shows the amount (% ID) of the Cupid-scFv antibody (24h before) + ²¹¹At-psyche-BR accumulated in the thyroid gland, the tumor-to-blood ratio, and the tumor-to-muscle ratio.

The clearance of the ²¹¹At-psyche-BR from the blood was 46.9±1.1% ID/g after 1 minute, 39.5±5.2% ID/g after 1 hour, 25.3±1.0% ID/g after 6 hours, and 8.3±0.5% ID/g after 24 hours. That is, the accumulation of the ²¹¹At-psyche-BR in the blood was decreased as time passed, and after 24 hours, about 92% of the amount of the ²¹¹At-psyche-BR administered was excreted.

Accumulation of the ²¹¹At-psyche-BR in the tumor was 0.6±0.1% ID/g after 1 minute, 3.1±0.7% ID/g after 1 hour, 9.3±0.6% ID/g after 6 hours, and 18.4±2.5% after 24 hours. That is, the 211At-psyche-BR in the tumor increased as time passed.

The tumor-to-blood ratio was 0.01±0.00 after 1 minute, 0.08±0.02 after 1 hour, 0.7±0.02 after 6 hours, and 2.23±0.41 after 24 hours.

The tumor-to-muscle ratio was 1.22±0.05 after 1 minute, 5.11±1.81 after 1 hour, 12.80±1.86 after 6 hours, and 25.07±3.68 after 24 hours.

Accumulation of the ²¹¹At-psyche-BR in the thyroid gland was low (0.15±0.05% ID after 24h), but an increasing tendency was observed as time passed.

Accumulation of the ²¹¹At-psyche-BR in the liver was 13.3±2.2% ID/g after 1 minute, 11.4±0.7% ID/g after 1 hour, 12.8±0.2% ID/g after 6 hours, and 7.8±0.9% after 24 hours. That is, the accumulation of the ²¹¹At-psyche-BR in the liver was high immediately after administration, and it was maintained at a certain level until 6 hours after the administration. After 24 hours, the amount of ²¹¹At-psyche-BR accumulated in the liver decreased to about half the 1 minute after the administration.

Accumulation of the ²¹¹At-psyche-BR in the spleen was 4.7±0.4% ID/g after 1 minute, 7.1±0.2% ID/g after 1 hour, 8.4±1.0% ID/g after 6 hours, and 5.5±1.1% after 24 hours. That is, the accumulation of the ²¹¹At-psyche-BR in the spleen increased as time passed, and it reached a peak 6 hours after the administration. After 24 hours, the amount of ²¹¹At-psyche-BR accumulated in the spleen was decreased to the level of 1 minute after the administration.

Accumulation of the ²¹¹At-psyche-BR in the kidney was 28.6±1.3% ID/g after 1 minute, 17.1±1.4% ID/g after 1 hour, 9.5±0.2% ID/g after 6 hours, and 5.4±0.5% after 24 hours.

Accumulation of the ²¹¹At-psyche-BR in the lung was 14.4±1.6% ID/g after 1 minute, 13.2±2.2% ID/g after 1 hour, 9.1±1.0% ID/g after 6 hours, and 5.3±0.1% after 24 hours. That is, accumulation of the ²¹¹At-psyche-BR in the lung was decreased as time passed.

### [Experiment 6: Studies regarding the pharmacokinetics of Cupid-scFv antibody (48h before) + ²¹¹At-psyche-BR in MKN-45 cancer-bearing mouse models]

### < Purpose >

The pharmacokinetics of ²¹¹At-psyche-BR to the Cupid-scFv antibody that had previously been administered (48h before) to MKN-45 cancer-bearing mouse models were examined.

### < Method >

The present experiment used human stomach cancer cells (MKN-45) expressing carcinoembryonic antigen (CEA). 9-week-old male immunodeficient mice (BALB/cSlc nu/nu) were used. MKN-45 cells (1 x 10⁷/0.1 ml) were transplanted into the mice's subcutis on the right shoulder dorsal side to produce cancer-bearing mouse models. The mice were divided into four groups when the tumor volume became a predetermined size (on Day 12 after the transplantation), and the Cupid-scFv antibody was administered 100 pmol/0.1 ml via the caudal vein of each mouse. Forty-eight hours after administration of the Cupid-scFv antibody, ²¹¹At-psyche-BR (about 0.25 MBq/100 ml) was administered via the caudal vein of each mouse. One minute, 1 hour, 6 hours, and 24 hours after administration of the ²¹¹At-psyche-BR, the mice were euthanized by collecting whole blood from the heart of each mouse under isoflurane anesthesia, and tumor tissues and normal tissues/organs were then excised. Normal tissues/organs: skin (lower right limb), muscle (right gastrocnemius muscle), heart, lung, spleen, pancreas, white fat (upper portion of right side testis), testis (both sides), stomach, stomach contents, small intestine (not including contents), large intestine (not including contents), liver, brown fat (interscapular), submandibular gland, thyroid gland (together with trachea), bone (right femur), cerebrum, eyeballs (both sides), bladder, and urine; the remaining residues were divided into three equal parts, and the three equal parts were defined to be residue-1, residue-2, and residue-3. After that, the weights of blood, plasma, urine, tumor, and normal tissues/organs were measured, and the radioactivity thereof was then measured using a γ counter. The amounts of the ²¹¹At-psyche-BR (radioactivity) accumulated in the blood, plasma, urine, tumor, and normal tissues/organs were calculated. That is to say, the rates (% ID/g) of the ²¹¹At-psyche-BR accumulated per unit weight (g) of the blood, plasma, urine, tumor, and normal tissues/organs, in the administered ²¹¹At-psyche-BR were shown. Regarding accumulation of the ²¹¹At-psyche-BR in the thyroid gland, since the weight of the thyroid gland could not be precisely measured, it was shown that the rate (% ID) of the ²¹¹At-psyche-BR accumulated in the thyroid gland in the administered ²¹¹At-psyche-BR.

### < Results >

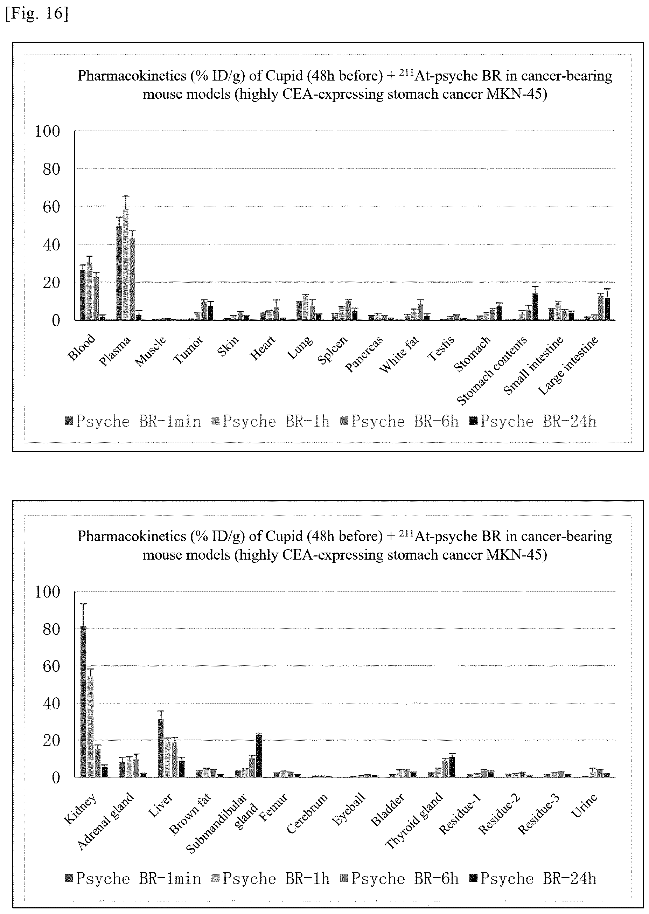

Fig. 16 shows the biodistribution (% ID/g) of the Cupid-scFv antibody (48h before) + ²¹¹At-psyche-BR.

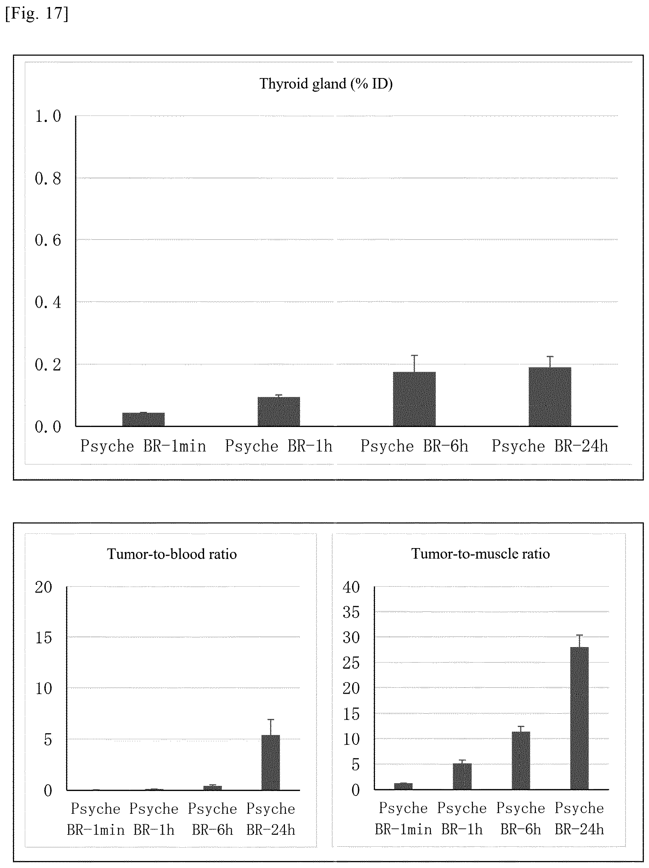

Fig. 17 shows the accumulation (% ID) of the Cupid-scFv antibody (48h before) + ²¹¹At-psyche-BR in the thyroid gland, the tumor-to-blood ratio, and the tumor-to-muscle balance.

The clearance of the ²¹¹At-psyche-BR from the blood was 26.4±2.7% ID/g after 1 minute, 30.6±3.2% ID/g after 1 hour, 22.7±2.7% ID/g after 6 hours, and 1.6±1.0% ID/g after 24 hours. That is, accumulation of the ²¹¹At-psyche-BR in the blood was maintained at a certain level until 6 hours after the administration, but after 24 hours, about 98% of the administered amount was excreted.

Accumulation of the ²¹¹At-psyche-BR in the tumor was 0.5±0.0% ID/g after 1 minute, 3.2±0.5% ID/g after 1 hour, 9.3±1.4% ID/g after 6 hours, and 7.4±2.3% after 24 hours. The accumulation of the ²¹¹At-psyche-BR in the tumor reached a peak 6 hours after the administration.

The tumor-to-blood ratio was 0.02±0.00 after 1 minute, 0.10±0.01 after 1 hour, 0.42±0.11 after 6 hours, and 5.3 9±1.5 6 after 24 hours.

The tumor-to-muscle ratio was 1.30±0.06 after 1 minute, 5.13±0.67 after 1 hour, 11.42±1.05 after 6 hours, and 27.98±2.34 after 24 hours.

Accumulation of the ²¹¹At-psyche-BR in the thyroid gland was low (0.19±0.03% ID after 24h), but an increasing tendency was observed as time passed.

Accumulation of the ²¹¹At-psyche-BR in the liver was 31.5±4.3% ID/g after 1 minute, 19.8±1.3% ID/g after 1 hour, 18.7±2.7% ID/g after 6 hours, and 8.9±1.7% after 24 hours. That is, accumulation of the ²¹¹At-psyche-BR in the liver was high immediately after administration, decreasing as time passed.

Accumulation of the ²¹¹At-psyche-BR in the spleen was 3.4±0.2% ID/g after 1 minute, 6.8±0.3% ID/g after 1 hour, 9.7±1.0% ID/g after 6 hours, and 4.6±1.6% after 24 hours. That is, the accumulation of the ²¹¹At-psyche-BR in the spleen increased as time passed, and it reached a peak 6 hours after the administration. After 24 hours, the amount of ²¹¹At-psyche-BR accumulated in the spleen was decreased to the level of 1 minute after the administration.

Accumulation of the ²¹¹At-psyche-BR in the kidney was 81.7±11.8% ID/g after 1 minute, 54.5±4.0% ID/g after 1 hour, 15.0±2.3% ID/g after 6 hours, and 5.6±1.0% after 24 hours.

Accumulation of the ²¹¹At-psyche-BR in the lung was 9.5±0.4% ID/g after 1 minute, 12.7±0.6% ID/g after 1 hour, 7.5±3.3% ID/g after 6 hours, and 2.9±0.3% after 24 hours.

### [Experiment 7: Studies regarding the pharmacokinetics of ²¹¹At-psyche-BR in MKN-45 cancer-bearing mouse models]

### < Purpose >

The pharmacokinetics of ²¹¹At-psyche-BR was examined in the absence of the Cupid-scFv antibody in MKN-45 cancer-bearing mouse models.

### < Method >

The present experiment used human stomach cancer cells (MKN-45) expressing carcinoembryonic antigen (CEA). 9-week-old male immunodeficient mice (BALB/cSlc nu/nu) were used. MKN-45 cells (1 x 10⁷/0.1 ml) were transplanted into the mice's subcutis on the right shoulder dorsal side to produce cancer-bearing mouse models. The mice were divided into four groups at the time the tumor volume became a predetermined size (on Day 19 after the transplantation), and ²¹¹At-psyche-BR was administered via the caudal vein of each mouse. One minute, 1 hour, 6 hours, and 24 hours after administration of the ²¹¹At-psyche-BR, the mice were euthanized by collecting whole blood from the heart of each mouse under isoflurane anesthesia, and tumor tissues and normal tissues/organs were then excised. Normal tissues/organs: muscle (right gastrocnemius muscle), heart, lung, spleen, pancreas, testis (both sides), stomach, stomach contents, small intestine (not including contents), large intestine (not including contents), liver, kidney, submandibular gland, thyroid gland (together with trachea), bone (right femur), and cerebrum. After that, the weights of blood, plasma, tumor, and normal tissues/organs were measured, and the radioactivity thereof was then measured using a γ counter. The amounts of the ²¹¹At-psyche-BR (radioactivity) accumulated in the blood, plasma, urine, tumor, and normal tissues/organs were calculated. That is to say, the rates (% ID/g) of the ²¹¹At-psyche-BR accumulated per unit weight (g) of the blood, plasma, urine, tumor, and normal tissues/organs, in the administered ²¹¹At-psyche-BR were shown. Regarding accumulation of the ²¹¹At-psyche-BR in the thyroid gland, since the weight of the thyroid gland could not be precisely measured, it was shown that the rate (% ID) of the ²¹¹At-psyche-BR accumulated in the thyroid gland in the administered ²¹¹At-psyche-BR.

### < Results >

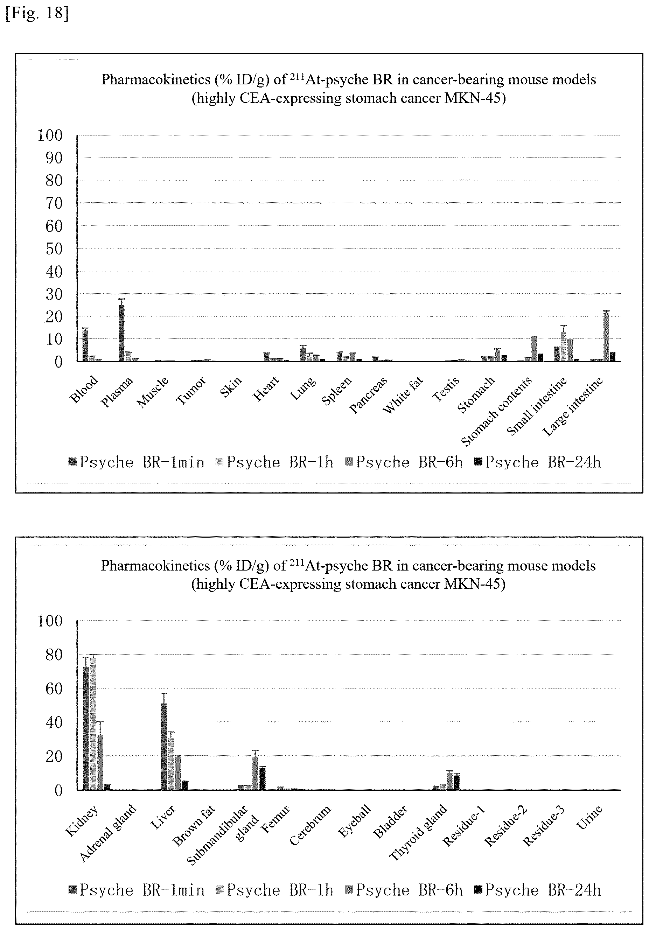

Fig. 18 shows the biodistribution (% ID/g) of ²¹¹At-psyche-BR.

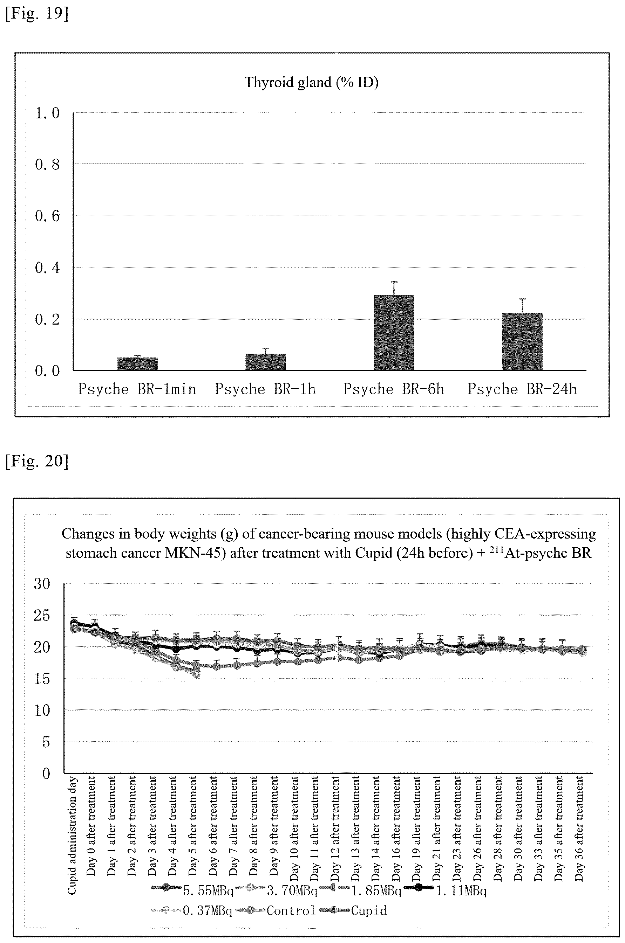

Fig. 19 shows the accumulation (% ID) of ²¹¹At-psyche-BR in the thyroid gland.

Accumulation of the ²¹¹At-psyche-B in the blood was 13.5±1.1% ID/g after 1 minute, 2.1±0.2% ID/g after 1 hour, 1.0±0.2% ID/g after 6 hours, and 0.2±0.04% ID/g after 24 hours. That is, the accumulation of the ²¹¹At-psyche-B in the blood was already low at the time point of 1 minute, and after 24 hours, it was decreased to about 0.2% of the administered amount. Thus, clearance from the blood was considerably rapid.

Accumulation of the ²¹¹At-psyche-B in the tumor was 0.3±0.04% ID/g after 1 minute, 0.4±0.02% ID/g after 1 hour, 0.8±0.09% ID/g after 6 hours, and 0.4±0.02% after 24 hours. That is, accumulation of the ²¹¹At-psyche-B in the tumor was hardly observed.

Accumulation of the ²¹¹At-psyche-B in the thyroid gland was low, and after 6 hours of administration, it was 0.29±0.05% ID.

Accumulation of the ²¹¹At-psyche-B in the kidney was 72.7±5.6% ID/g after 1 minute, 77.9±2.1% ID/g after 1 hour, 32.1±8.2% ID/g after 6 hours, and 3.0±0.1% after 24 hours. That is, the ²¹¹At-psyche-B was excreted from the kidney immediately after administration; after 24 hours, the amount of ²¹¹At-psyche-B accumulated in the kidney was decreased to 3.0% of the administered amount.

Accumulation of the ²¹¹At-psyche-B in the liver was 51.1±5.9% ID/g after 1 minute, 30.8±3.3% ID/g after 1 hour, 19.6±0.5% ID/g after 6 hours, and 5.0±0.2% after 24 hours. That is, the ²¹¹At-psyche-B in the liver was high immediately after administration. Then, as time passed, the amount of ²¹¹At-psyche-B accumulated in the liver decreased.

### [Experiment 8: Evaluation of therapeutic effects and toxicity of Cupid-scFv antibody (24h before) + ²¹¹At-psyche-BR in MKN-45 cancer-bearing mouse models]

### < Purpose >

The therapeutic effects and toxicity of the Cupid-scFv antibody (24h before) + ²¹¹At-psyche-BR in MKN-45 cancer-bearing mouse models were evaluated over time.

### < Method >

The present experiment used human stomach cancer cells (MKN-45) expressing carcinoembryonic antigen (CEA). 9-week-old male immunodeficient mice (BALB/cSlc nu/nu) were used. MKN-45 cells (5 x 10⁶/0.1 ml) were transplanted into the mice's subcutis on the right shoulder dorsal side to produce cancer-bearing mouse models. At the time point in which the tumor volume became a predetermined size (on Day 8 after the transplantation), the mice were divided into seven groups, which were for use in the evaluation of the therapeutic effects, and a biodistribution group (6 hours) of cancer-bearing mouse models having this tumor size. The Cupid-scFv antibody was administered 100 pmol/0.1 ml via the caudal vein of each mouse. Twenty-four hours after administration of the Cupid-scFv antibody, ²¹¹At-psyche-BR was administered via the caudal vein of each mouse in an amount of 5.55 MBq, 3.70 MBq, 1.85 MBq, 1.11 MBq, or 0.37 MBq.
To a control group, a solvent for the ²¹¹At-psyche-BR was administered (solvent group). A group to which only cupid was administered was defined as a Cupid group. The mice's general states were observed and recorded immediately after administration of the ²¹¹At-psyche-BR and at 3 hours after the administration.
Moreover, until Day 14, after administration of the ²¹¹At-psyche-BR, observation of the general states and the measurement of the body weight was carried out every day. After Day 14 of the administration of the ²¹¹At-psyche-BR, observation of the general states and the measurement of the body weight was carried out every other day. The tumor size was measured every other day after the treatment with the ²¹¹At-psyche-BR. On Day 36, after the treatment with the ²¹¹At-psyche-BR, observation of the general states, the measurement of the body weight, and the measurement of the tumor size were carried out, and the mice were then euthanized by collecting whole blood from the heart of each mouse under isoflurane anesthesia. Tumor tissues and major tissues/organs were excised from the mice, weighed, and immobilized with formalin.

### < Results >

Fig. 20 shows changes in the mice' body weights (g) over time.

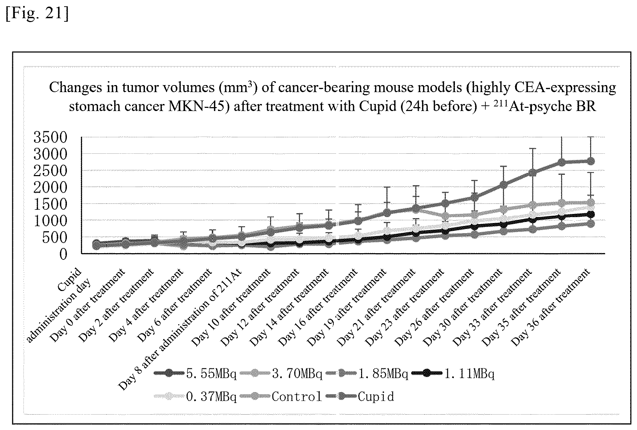

Fig. 21 shows changes over time in the tumor volumes (mm³).

In the 5.55 MBq and 3.70 MBq ²¹¹At-psyche-BR administration groups, after administration of the ²¹¹At-psyche-BR, the body weight was reduced as time passed. On Day 5, since general states and body weight reduction became severe, the mice were subjected to urgent euthanasia, and the experiment was terminated.

Also, in the 1.85 MBq ²¹¹At-psyche-BR administration group, general states and body weight reduction became significantly severe on Day 5. However, observation was continued without being subjected to euthanasia. On and after Day 6, the recovery of the general states and the body weight reduction were observed, and From Day 19, the mice wholly recovered.

In the 1.11 MBq and 0.37 MBq ²¹¹At-psyche-BR administration groups, general states and body weight changes were at almost the same levels as in the control group.

Compared with the control group, tumor growth was suppressed by the treatment with 1.85 MBq, 1.11 MBq, and 0.37 MBq 211At-psyche-BR until Day 10 of the treatment tumor volume. After that, the tumor volume increased as time passed, but the tumor growth was suppressed in an amount-dependent manner compared with the control group.

### [Experiment 9: Studies regarding pharmacokinetics (6h) of Cupid-scFv antibody (24h before) + ²¹¹At-psyche-BR in MKN-45 cancer-bearing mouse models]

### < Purpose >

The pharmacokinetics (6h) of ²¹¹At-psyche-BR to the Cupid-scFv antibody that had previously been administered (24h before) to MKN-45 cancer-bearing mouse models were examined.

### < Method >

The present experiment used human stomach cancer cells (MKN-45) expressing carcinoembryonic antigen (CEA). 9-week-old male immunodeficient mice (BALB/cSlc nu/nu) were used. MKN-45 cells (1 x 10⁷/0.1 ml) were transplanted into the mice's subcutis on the right shoulder dorsal side to produce cancer-bearing mouse models. When the tumor volume became a predetermined size (on Day 8 after the transplantation), the Cupid-scFv antibody was administered 100 pmol/0.1 ml via the caudal vein of each mouse. Twenty-four hours after administration of the Cupid-scFv antibody, ²¹¹At-psyche-BR (about 0.30 MBq/100 ml) was administered via the caudal vein of each mouse. Six hours after administration of the ²¹¹At-psyche-BR, the mice were euthanized by collecting whole blood from the heart of each mouse under isoflurane anesthesia, and tumor tissues and normal tissues/organs were then excised. Normal tissues/organs: skin (lower right limb), muscle (right gastrocnemius muscle), heart, lung, spleen, pancreas, white fat (upper portion of right side testis), testis (both sides), stomach, stomach contents, small intestine (not including contents), large intestine (not including contents), liver, brown fat (interscapular), submandibular gland, thyroid gland (together with trachea), bone (right femur), cerebrum, eyeballs (both sides), bladder, and urine; the remaining residues were divided into three equal parts, and the three equal parts were defined to be residue-1, residue-2, and residue-3. After that, the weights of blood, plasma, urine, tumor, and normal tissues/organs were measured, and the radioactivity thereof was then measured using a γ counter. The amounts of the ²¹¹At-psyche-BR (radioactivity) accumulated in the blood, plasma, urine, tumor, and normal tissues/organs were calculated. That is to say, the rates (% ID/g) of the ²¹¹At-psyche-BR accumulated per unit weight (g) of the blood, plasma, urine, tumor, and normal tissues/organs, in the administered ²¹¹At-psyche-BR were shown. Regarding accumulation of the ²¹¹At-psyche-BR in the thyroid gland, since the weight of the thyroid gland could not be precisely measured, it was shown that the rate (% ID) of the ²¹¹At-psyche-BR accumulated in the thyroid gland in the administered ²¹¹At-psyche-BR.

### < Results >

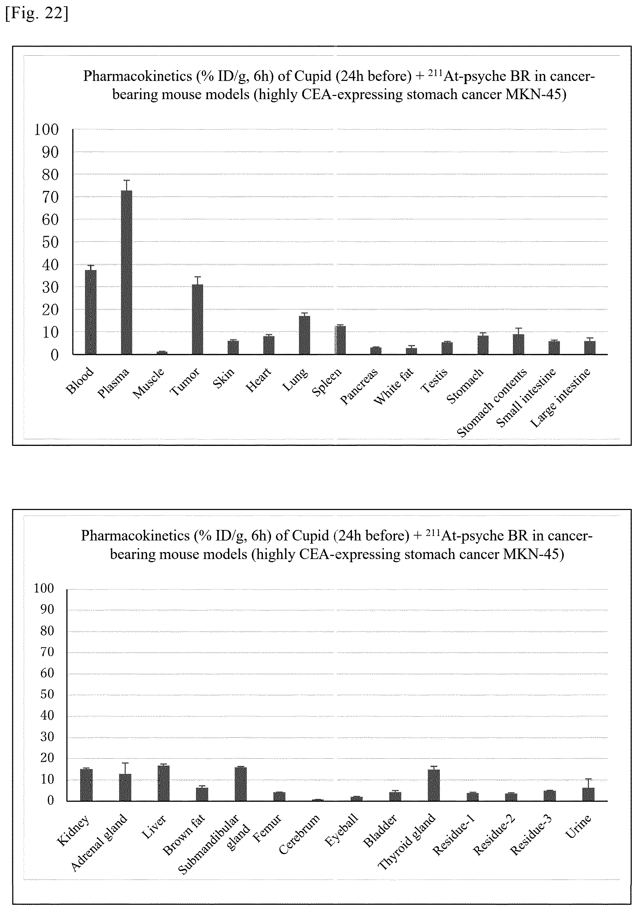

Fig. 22 shows the pharmacokinetics (% ID/g, 6h) of the Cupid-scFv antibody (24h before) + ²¹¹At-psyche-BR.

Accumulating the ²¹¹At-psyche-BR in the tumor was high, and it was 31.0±3.4 % ID/g.

### [Experiment 10: Evaluation of therapeutic effects and toxicity of Cupid-scFv antibody (24h before) + ²¹¹At-psyche-B in MKN-45 cancer-bearing mouse models]

### < Purpose >

The therapeutic effects and toxicity of the Cupid-scFv antibody (24h before) + ²¹¹At-psyche-B in MKN-45 cancer-bearing mouse models were evaluated over time.

### < Method >

The present experiment used human stomach cancer cells (MKN-45) expressing carcinoembryonic antigen (CEA). 9-week-old male immunodeficient mice (BALB/cSlc nu/nu) were used. MKN-45 cells (5 x 10⁶/0.1 ml) were transplanted into the mice's subcutis on the right shoulder dorsal side to produce cancer-bearing mouse models. At the time point in which the tumor volume became a predetermined size (on Day 8 after the transplantation), the mice were divided into six groups, which were for use in the evaluation of the therapeutic effects, and a biodistribution group (6 hours) of cancer-bearing mouse models having this tumor size. The Cupid-scFv antibody was administered 100 pmol/0.1 ml via the caudal vein of each mouse. Twenty-four hours after administration of the Cupid-scFv antibody, ²¹¹At-psyche-B was administered via the caudal vein of each mouse in an amount of 3.70 MBq, 1.85 MBq, 1.11 MBq, or 0.37 MBq.
In the present experiment, a 5.55 MBq administration group was not established. To a control group, a solvent for the ²¹¹At-psyche-B was administered (solvent group). A group to which only cupid was administered was defined as a Cupid group. The mice's general states were observed and recorded immediately after administration of the ²¹¹At-psyche-B and at 3 hours after the administration. Moreover, until Day 14, after administration of the ²¹¹At-psyche-B, observation of the general states and the measurement of the body weight was carried out every day. After Day 14 of the administration of the ²¹¹At-psyche-BR, observation of the general states and the measurement of the body weight was carried out every other day. The tumor size was measured every other day after the treatment with the ²¹¹At-psyche-B. On Day 36, after the treatment with the ²¹¹At-psyche-B, observation of the general states, the measurement of the body weight, and the measurement of the tumor size were carried out, and the mice were then euthanized by collecting whole blood from the heart of each mouse under isoflurane anesthesia. Tumor tissues and major tissues/organs were excised from the mice, weighed, and immobilized with formalin.

### < Results >

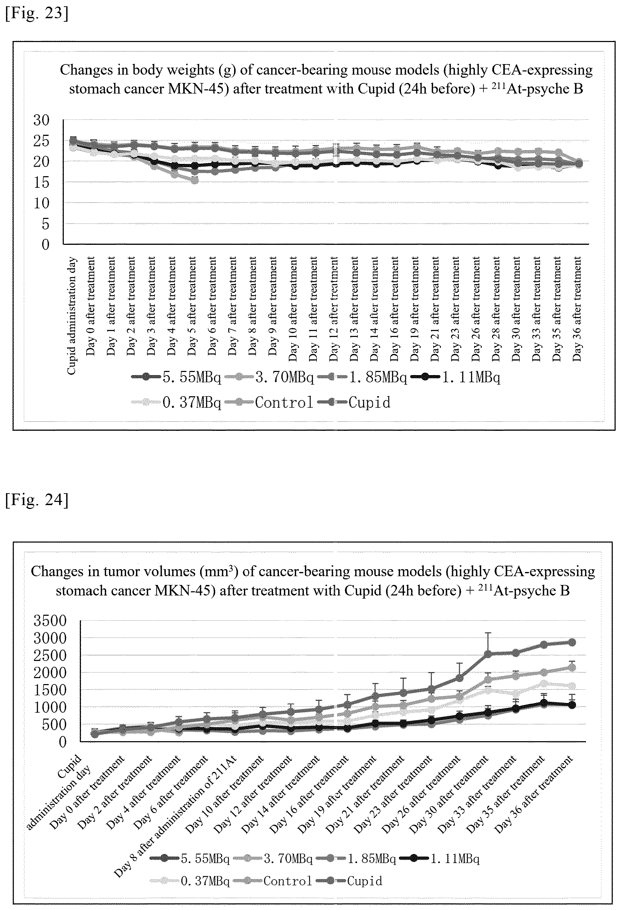

Fig. 23 shows changes in the mice' body weights (g) over time.

Fig. 24 shows changes over time in the tumor volumes (mm³).

In the 3.70 MBq ²¹¹At-psyche-B administration groups, after administration of the ²¹¹At-psyche-B, the body weight was reduced as time passed. On Day 5, since general states and body weight reduction became severe, the mice were subjected to urgent euthanasia, and the experiment was terminated.

Also, in the 1.85 MBq ²¹¹At-psyche-B administration group, general states and body weight reduction became significantly severe on Day 5. However, observation was continued without being subjected to euthanasia. On and after Day 6, red spots were dispersed on the skin, and on Day 10, one mouse died. Such red spots on the skin continued until Day 12. On and after Day 12, the general states of two mice were recovered, and on Day 26, the mice were completely recovered.

In the 1.11 MBq ²¹¹At-psyche-B administration group, the body weight was slightly reduced until Day 5 and somewhat recovered. However, the body weight was not recovered to the same level as the control groups.

In the 0.37 MBq ²¹¹At-psyche-B administration group, body weight reduction was lower than that in the 1.11 MBq ²¹¹At-psyche-B administration group. On and after Day 10, the body weight reduction was the same as in the 1.11 MBq ²¹¹At-psyche-B administration group.

Compared with the control group, tumor growth was suppressed by the treatment with 1.85 MBq, 1.11 MBq, and 0.37 MBq 211At-psyche-B until Day 10 of the treatment volume. After that, the tumor volume increased as time passed, but the tumor growth was suppressed in an amount-dependent manner compared with the control group.

### [Experiment 11: Studies of pharmacokinetics (6h) of Cupid-scFv antibody (24h before) + ²¹¹At-psyche-B in MKN-45 cancer-bearing mouse models]

### < Purpose >

The pharmacokinetics (6h) of ²¹¹At-psyche-B to the Cupid-scFv antibody that had previously been administered (24h before) to MKN-45 cancer-bearing mouse models were examined.

### < Method >

The present experiment used human stomach cancer cells (MKN-45) expressing carcinoembryonic antigen (CEA). 9-week-old male immunodeficient mice (BALB/cSlc nu/nu) were used. MKN-45 cells (1 x 10⁷/0.1 ml) were transplanted into the mice's subcutis on the right shoulder dorsal side to produce cancer-bearing mouse models. When the tumor volume became a predetermined size (on Day 8 after the transplantation), the Cupid-scFv antibody was administered 100 pmol/0.1 ml via the caudal vein of each mouse. Twenty-four hours after administration of the Cupid-scFv antibody, ²¹¹At-psyche-B (about 0.30 MBq/100 ml) was administered via the caudal vein of each mouse. Six hours after administration of the ²¹¹At-psyche-B, the mice were euthanized by collecting whole blood from the heart of each mouse under isoflurane anesthesia, and tumor tissues and normal tissues/organs were then excised. Normal tissues/organs: skin (lower right limb), muscle (right gastrocnemius muscle), heart, lung, spleen, pancreas, white fat (upper portion of right side testis), testis (both sides), stomach, stomach contents, small intestine (not including contents), large intestine (not including contents), liver, brown fat (interscapular), submandibular gland, thyroid gland (together with trachea), bone (right femur), cerebrum, eyeballs (both sides), bladder, and urine; the remaining residues were divided into three equal parts, and the three equal parts were defined to be residue-1, residue-2, and residue-3. After that, the weights of blood, plasma, urine, tumor, and normal tissues/organs were measured, and the radioactivity thereof was then measured using a γ counter. The amounts of the ²¹¹At-psyche-B (radioactivity) accumulated in the blood, plasma, urine, tumor, and normal tissues/organs were calculated. That is to say, the rates (% ID/g) of the ²¹¹At-psyche-B accumulated per unit weight (g) of the blood, plasma, urine, tumor, and normal tissues/organs, in the administered ²¹¹At-psyche-B, were shown. Regarding accumulation of the ²¹¹At-psyche-B in the thyroid gland, since the weight of the thyroid gland could not be precisely measured, it was shown that the rate (% ID) of the ²¹¹At-psyche-B accumulated in the thyroid gland in the administered ²¹¹At-psyche-B.

### < Results >

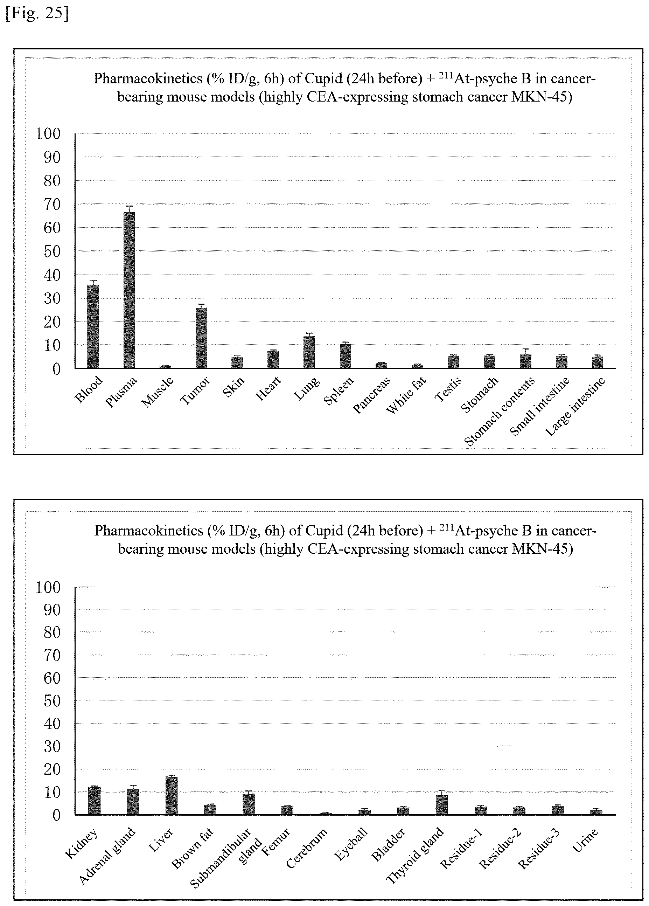

Fig. 25 shows the pharmacokinetics (% ID/g, 6h) of the Cupid-scFv antibody (24h before) + ²¹¹At-psyche-B.

Accumulation of the ²¹¹At-psyche-B in the tumor was high, and it was 25.8±1.5 % ID/g.

## Claims

1. A compound represented by the following formula (1) or a salt thereof: 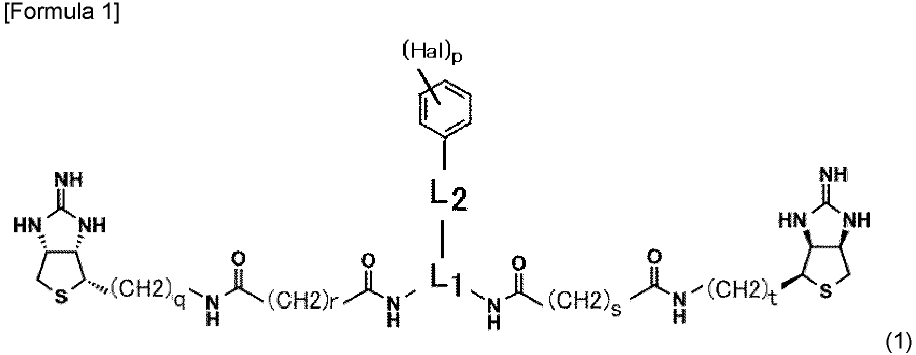 wherein
L₁ represents a trivalent linking group,
L₂ represents a divalent linking group,
Hal represents a halogen,
p represents an integer of 1 to 5, and
q, r, s, and t each independently represent an integer of 1 to 8.

2. The compound according to claim 1 or a salt thereof, wherein L₁ represents the following:

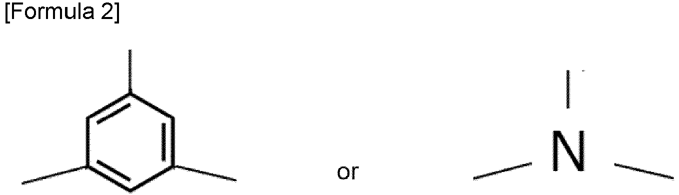

3. The compound according to claim 1 or 2, or a salt thereof, wherein L₂ represents a divalent linking group consisting of a combination of groups selected from -CONH-, -NHCO-, -COO-, -OCO-, -CO-, -O-, and an alkylene group containing 1 to 10 carbon atoms.

4. The compound according to any one of claims 1 to 3, or a salt thereof, which is a compound represented by the following formula (2) or a salt thereof: 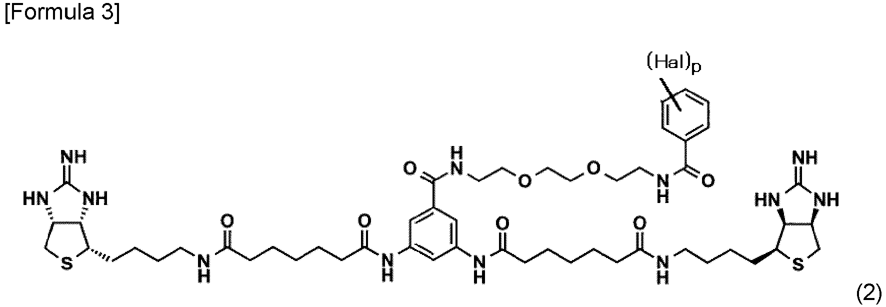 wherein individual symbols are as defined in claim 1.

5. The compound according to any one of claims 1 to 4, or a salt thereof, wherein Hal is I, ¹²⁵I, or ²¹¹At.

6. The compound according to any one of claims 1 to 5, or a salt thereof, wherein p is 1.

7. The compound according to claim 1, or a salt thereof, represented by the following formulae:

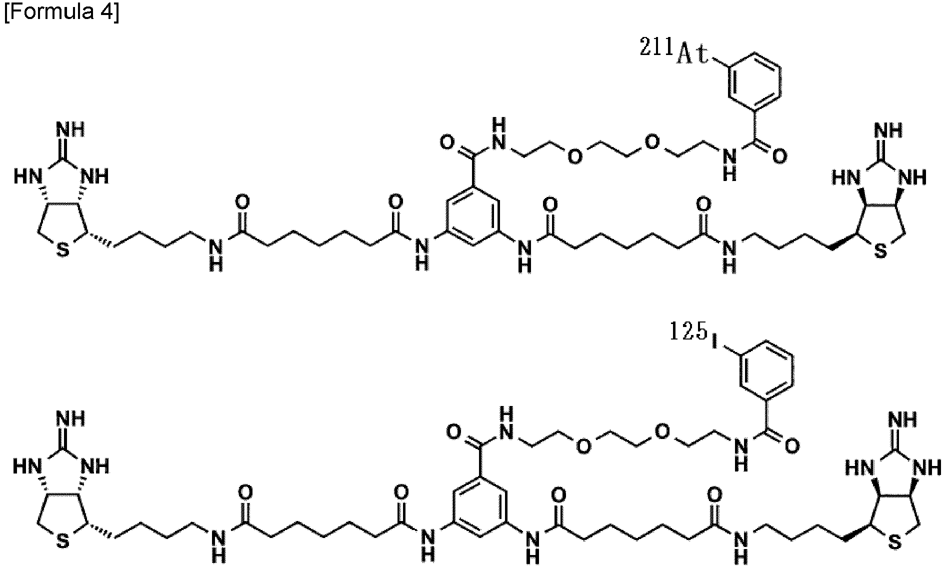

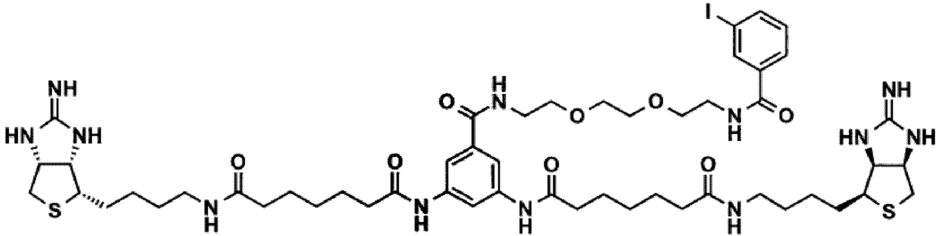

8. A kit for use in the treatment or diagnosis, comprising: (1) the compound according to any one of claims 1 to 7; and (b) a mutant streptavidin-molecular probe conjugate obtained by binding a molecular probe to a mutant streptavidin comprising the amino acid sequence as outlined in SEQ ID NO: 1 (provided that the amino acid sequence consisting of six histidine residues at the C-terminus may be partially or entirely deleted).

## Patentansprüche

1. Eine durch die folgende Formel (1) dargestellte Verbindung oder ein Salz davon: 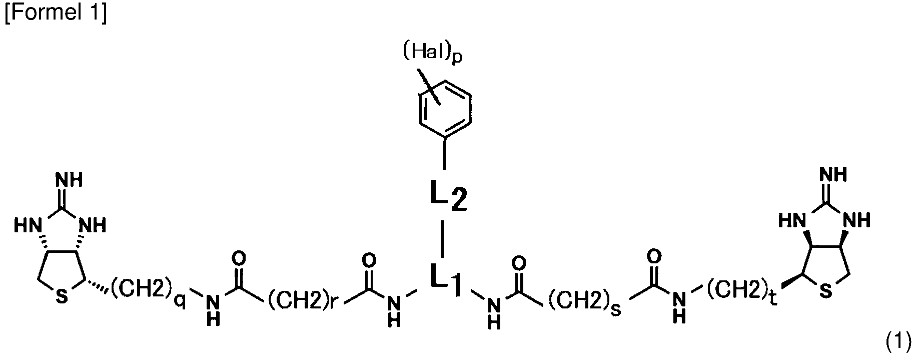 wobei
L₁ eine dreiwertige Verbindungsgruppe darstellt,
L₂ eine zweiwertige Verbindungsgruppe darstellt,
Hal ein Halogen darstellt,
p eine ganze Zahl von 1 bis 5 darstellt, und
q, r, s, und t jeweils unabhängig voneinander eine ganze Zahl von 1 bis 8 darstellt.

2. Die Verbindung gemäß Anspruch 1 oder ein Salz davon, wobei L₁ die folgenden Gruppen darstellt:

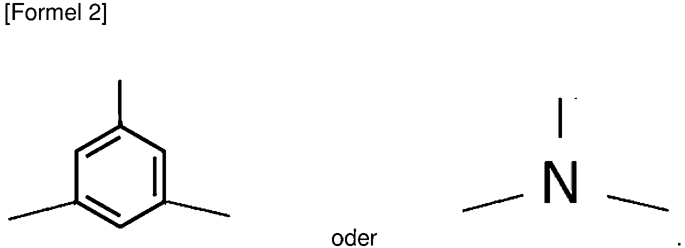

3. Die Verbindung gemäß Anspruch 1, oder ein Salz davon, wobei L₂ eine zweiwertige Verbindungsgruppe darstellt bestehend aus einer Kombination von Gruppen ausgewählt aus -CONH-, -NHCO-, -COO-, -OCO-, -CO-, -O-, und einer Alkylengruppe enthaltend 1 bis 10 Kohlenstoffatome.

4. Die Verbindung gemäß einem der Ansprüche 1 bis 3, oder ein Salz davon, welches eine durch die folgende Formel (2) dargestellte Verbindung oder ein Salz davon ist: 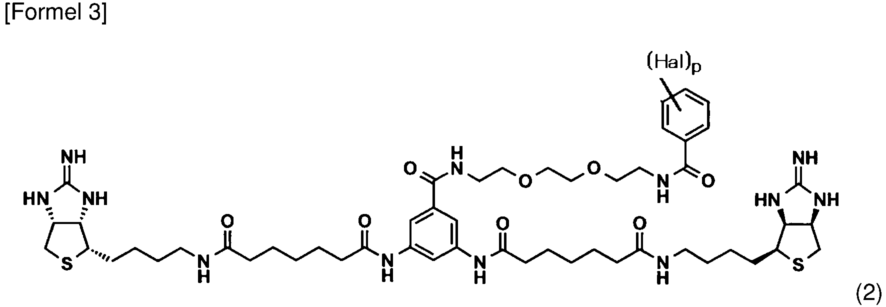 wobei die individuellen Symbole wie in Anspruch 1 definiert sind.

5. Die Verbindung gemäß einem der Ansprüche 1 bis 4, oder ein Salz davon, wobei Hal I, ¹²⁵I, oder ²¹¹At ist.

6. Die Verbindung gemäß einem der Ansprüche 1 bis 5, oder ein Salz davon, wobei p 1 ist.

7. Die Verbindung gemäß Anspruch 1, oder ein Salz davon, dargestellt durch die folgenden Formeln:

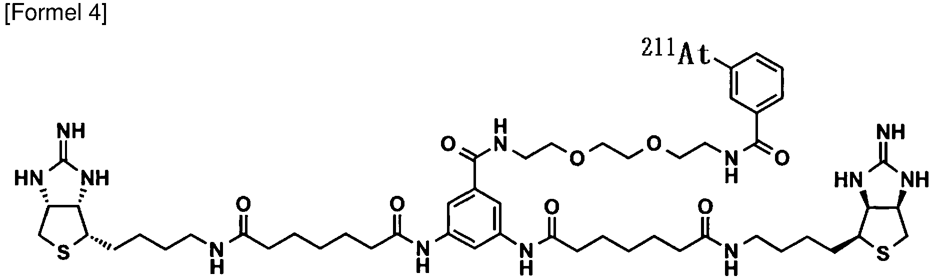

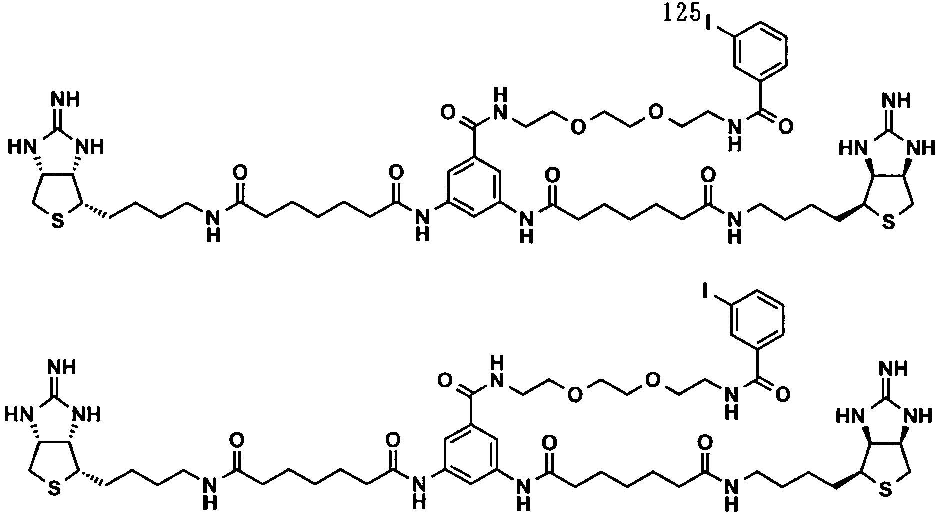

8. Ein Kit zur Verwendung in der Behandlung oder Diagnose, umfassend: (1) die Verbindung gemäß einem der Ansprüche 1 bis 7; und (b) ein Konjukat einer molekularen Sonde eines mutierten Streptavidins, das erhalten wird durch Bindung einer molekularen Sonde an ein mutiertes Streptavidin umfassend die in SEQ ID Nr.: 1 dargestellte Aminosäuresequenz (mit der Maßgabe, dass die Aminosäuresequenz, die aus sechs Histidin-Resten am C-Terminus besteht, ganz oder teilweise entfernt sein kann).

## Revendications

1. Composé représenté par la formule (1) suivante ou un sel de celui-ci : 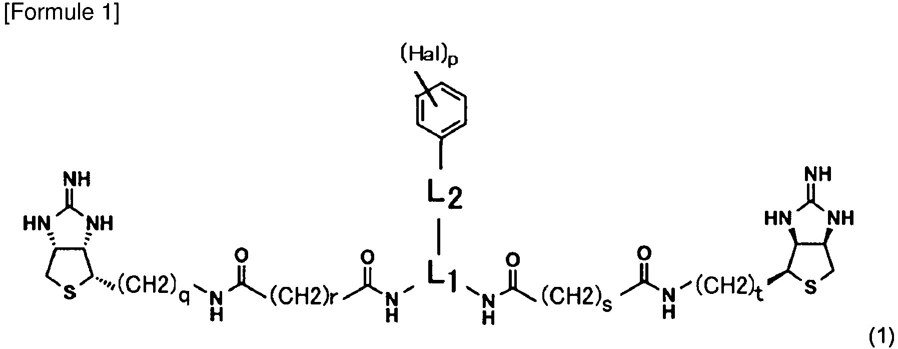 dans lequel
L₁ représente un groupe de liaison trivalent,
L₂ représente un groupe de liaison divalent,
Hal représente un halogène,
p représente un nombre entier compris entre 1 et 5, et
q, r, s et t représentent chacun indépendamment un nombre entier compris entre 1 et 8.

2. Composé selon la revendication 1 ou un sel de celui-ci, dans lequel L₁ représente ce qui suit :

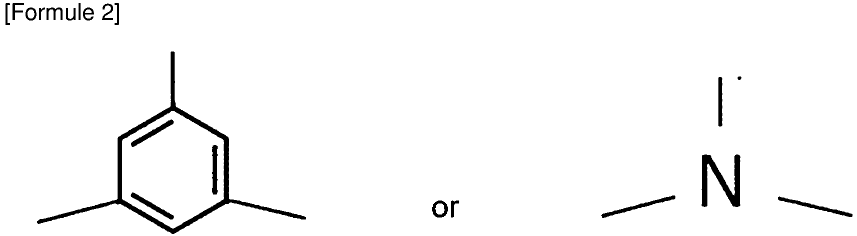

3. Composé selon la revendication 1 ou 2, ou un sel de celui-ci, dans lequel L₂ représente un groupe de liaison divalent constitué d'une combinaison de groupes choisis parmi -CONH-, -NHCO-, -COO-, -OCO-, -CO-, -O- et un groupe alkylène contenant 1 à 10 atomes de carbone.

4. Composé selon l'une quelconque des revendications 1 à 3, ou un sel de celui-ci, qui est un composé représenté par la formule (2) suivante ou un sel de celui-ci : 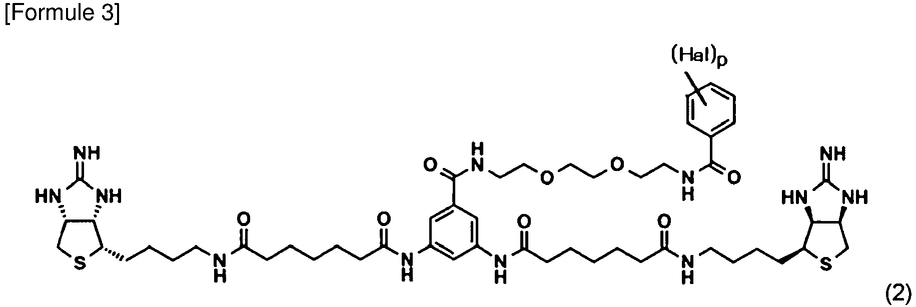 dans lequel les symboles individuels sont tels que définis dans la revendication 1.

5. Composé selon l'une quelconque des revendications 1 à 4, ou un sel de celui-ci, dans lequel Hal représente I, ¹²⁵I ou ²¹¹At.

6. Composé selon l'une quelconque des revendications 1 à 5, ou un sel de celui-ci, dans lequel p est égal à 1.

7. Composé selon la revendication 1, ou un sel de celui-ci, représenté par les formules suivantes :

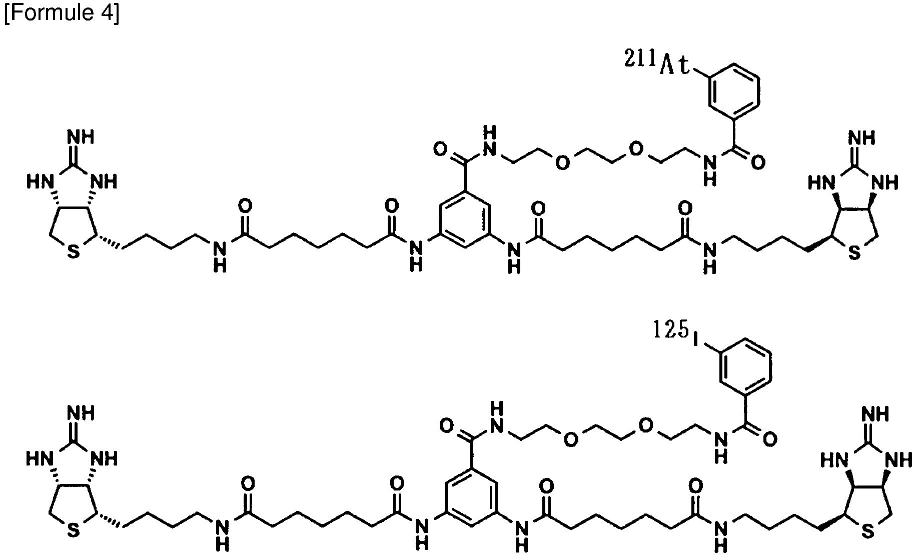

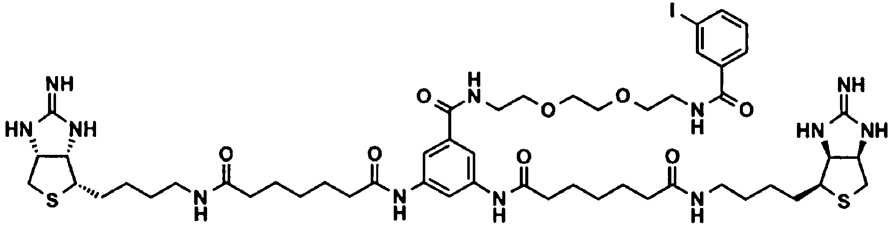

8. Kit destiné à être utilisé dans le traitement ou le diagnostic, comprenant : (1) le composé selon l'une quelconque des revendications 1 à 7 ; et (b) un conjugué de streptavidine mutante et de sonde moléculaire obtenu en liant une sonde moléculaire à une streptavidine mutante comprenant la séquence d'acides aminés décrite dans SEQ ID NO: 1 (à condition que la séquence d'acides aminés constituée de six résidus histidine à l'extrémité C-terminale puisse être partiellement ou entièrement supprimée).
